# EUROPEAN PATENT APPLICATION

(11) **EP 3 479 765 A1**
(43) Date of publication of application: **08.05.2019**
(21) Application number: 17819590.5
(22) Date of filing: 03.04.2017
(51) Int. Cl.: A61B 5/026, A61B 5/0295, A61B 5/107, A61B 5/11

(54) **INFORMATION PROCESSING DEVICE, INFORMATION PROCESSING METHOD, AND PROGRAM**

(30) Priority: 01.07.2016 JP 2016131505
(71) Applicant: Sony Corporation, Tokyo 108-0075 (JP)
(72) Inventor: MIYASHITA, Ken, Tokyo 108-0075 (JP); WAKITA, Yoshihiro, Tokyo 108-0075 (JP); ALTINTAS, Ersin, Tokyo 108-0075 (JP); KAWAMOTO, Yohei, Tokyo 108-0075 (JP)
(74) Representative: MFG Patentanwälte Meyer-Wildhagen Meggle-Freund Gerhard PartG mbB
(86) International application number: PCT/JP2017/014000
(87) International publication number: WO 2018/003221

(57) **Abstract**

To propose an information processing apparatus which is capable of encouraging behaviors that have an effect appropriate for the body of a measured person in accordance with the physical condition of the measured person.

There is proposed an information processing apparatus, including: a comparing section configured to perform a comparison using a blood flow velocity of a measured person in a predetermined state and a blood flow velocity of the measured person in a state other than the predetermined state; and an information presenting section configured to present information corresponding to a comparison result of the comparing section.

## Description

### Technical Field

The present disclosure relates to an information processing apparatus, an information processing method, and a program.

### Background Art

Techniques for measuring information related to blood flow such as a pulse and a blood flow velocity are frequently used in the field of medicine and the like. As an example of an apparatus for measuring a pulse and a blood flow velocity, a blood flowmeter can be mentioned. A blood flowmeter can be constantly installed on a measured person without giving discomfort, pain, or the like to the measured person and easily measure the pulse and the blood flow velocity. For example, an example of a blood flowmeter is disclosed in Patent Literature 1.

### Citation List

### Patent Literature

- Patent Literature 1:: JP 2013-146371A

### Disclosure of Invention

### Technical Problem

If information of a physical condition obtained from the blood flow velocity measured by a blood flowmeter can be fed back to the measured person or the like, it is possible to remind the person of behaviors that have an effect appropriate for the body of the measured person, leading to the health enhancement of the measured person.

In this regard, in light of the foregoing, the present disclosure proposes an information processing apparatus, an information processing method, and a program which are capable of encouraging behaviors that have an effect appropriate for the body of a measured person in accordance with the physical condition of the measured person.

### Solution to Problem

According to the present disclosure, there is provided an information processing apparatus, including: a comparing section configured to perform a comparison using a blood flow velocity of a measured person in a predetermined state and a blood flow velocity of the measured person in a state other than the predetermined state; and an information presenting section configured to present information corresponding to a comparison result of the comparing section.

In addition, according to the present disclosure, there is provided an information processing method, including: performing a comparison using a blood flow velocity of a measured person in a predetermined state and a blood flow velocity of the measured person in a state other than the predetermined state; and presenting information corresponding to a comparison result obtained through the comparison.

Further, according to the present disclosure, there is provided a program causing a computer to implement: a function of performing a comparison using a blood flow velocity of a measured person in a predetermined state and a blood flow velocity of the measured person in a state other than the predetermined state; and a function of presenting information corresponding to a result of the comparison.

### Advantageous Effects of Invention

As explained above, according to the present disclosure, it is possible to encourage behaviors that have an effect appropriate for the body of the measured person in accordance with the physical condition of the measured person.

Note that the effects described above are not necessarily limitative. With or in the place of the above effects, there may be achieved any one of the effects described in this specification or other effects that may be grasped from this specification.

### Brief Description of Drawings

FIG. 1 is an explanatory diagram for describing a DLS technique applied to an embodiment of the present disclosure.
FIG. 2 is an explanatory diagram for describing a PPG technique applied to an embodiment of the present disclosure.
FIG. 3 is a system diagram illustrating a configuration of an information processing system 1 according to an embodiment of the present disclosure.
FIG. 4 is a diagram illustrating an example of a form of a measuring module 10 according to an embodiment of the present disclosure (1/2).
FIG. 5 is an explanatory diagram for describing a form when the measuring module 10 illustrated in FIG. 4 is installed.
FIG. 6 is an explanatory diagram for describing an installation example of the measuring module 10 illustrated in FIG. 4.
FIG. 7 is a diagram illustrating an example of a form of a measuring module 10 according to an embodiment of the present disclosure (2/2).
FIG. 8 is an explanatory diagram for describing an installation example of the measuring module 10 illustrated in FIG. 7.
FIG. 9 is a system diagram illustrating a configuration of an information processing system 1 according to a first embodiment of the present disclosure.
FIG. 10 is a flowchart of an information processing method according to the first embodiment of the present disclosure.
FIG. 11 is an explanatory diagram for describing an installation example of a measuring module 10 according to a second embodiment of the present disclosure.
FIG. 12 is a flowchart illustrating an information processing method according to the second embodiment of the present disclosure.
FIG. 13 is an explanatory diagram for describing an example of a display screen 700 according to the second embodiment of the present disclosure.
FIG. 14 is an explanatory diagram for describing an example of a display screen 702 according to the second embodiment of the present disclosure.
FIG. 15 is an explanatory diagram for describing an example of a display screen 704 according to the second embodiment of the present disclosure.
FIG. 16 is an explanatory diagram for describing an example of a display screen 706 according to the second embodiment of the present disclosure.
FIG. 17 is an explanatory diagram for describing an example of a display screen 708 according to the second embodiment of the present disclosure.
FIG. 18 is a flowchart of an information processing method according to a third embodiment of the present disclosure.
FIG. 19 is an explanatory diagram for describing an example of a display screen 750 according to the third embodiment of the present disclosure.
FIG. 20 is an explanatory diagram for describing an example of a display screen 752 according to the third embodiment of the present disclosure.
FIG. 21 is an explanatory diagram for describing an example of a display screen 754 according to the third embodiment of the present disclosure.
FIG. 22 is an explanatory diagram for describing an example of a display screen 756 according to the third embodiment of the present disclosure.
FIG. 23 is an explanatory diagram for describing an example of a display screen 758 according to the third embodiment of the present disclosure.
FIG. 24 is an explanatory diagram for describing a fourth embodiment of the present disclosure.
FIG. 25 is an explanatory diagram for describing a configuration of a measuring unit of a measuring module according to a comparative example.
FIG. 26 is an explanatory diagram for describing a configuration of a measuring unit of a measuring module according to a fifth embodiment of the present disclosure.
FIG. 27 is an explanatory diagram for describing a configuration of a measuring unit of a measuring module according to a modified example of the fifth embodiment of the present disclosure.
FIG. 28 is an explanatory diagram for describing a measurement method according to a comparative example.
FIG. 29 is an explanatory diagram for describing a measurement method according to a sixth embodiment of the present disclosure.
FIG. 30 is an explanatory diagram for describing a measurement method in accordance with a first modified example of the sixth embodiment of the present disclosure.
FIG. 31 is an explanatory diagram for describing a measurement method in accordance with a second modified example of the sixth embodiment of the present disclosure.
FIG. 32 is a block diagram illustrating a configuration of an information processing apparatus according to an embodiment of the present disclosure.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Further, in the following description, a measurement subject whose blood flow velocity is measured in a state in which he or she wears a measuring module according to an embodiment of the present disclosure to be described below on his or her arm or the like is referred to as a measured person. Further, in the following description, a user who uses an information processing system according to an embodiment of the present disclosure to be described below is referred to as a user, and the above-mentioned measuring person is also the user.

The description will proceed in the following order.
1. Blood flow velocity measurement method
2. Information processing system according to embodiment of the present disclosure
3. Information processing method according to first embodiment of present disclosure
4. Information processing method according to second embodiment of present disclosure
5. Information processing method according to third embodiment of present disclosure
6. Information processing method according to fourth embodiment of present disclosure
7. Information processing method according to fifth embodiment of present disclosure
8. Measurement method according to sixth embodiment of present disclosure
9. Hardware configuration
10. Supplement

### <<1. Blood flow velocity measurement method>>

First, a blood flow velocity measurement method used in an embodiment of the present disclosure will be briefly described. In the following description, the blood flow velocity indicates a velocity of blood flowing in one or more blood vessels in a measurement region serving as a measurement target or a blood flow rate per unit time carried by one or more blood vessels in a measurement region. Examples of the blood flow velocity measurement method applicable to an embodiment of the present disclosure include a dynamic light scattering (DLS) technique and a photoplethysmography (PPG) technique. However, the blood flow velocity measurement method applicable to an embodiment of the present disclosure is not limited to the PPG technique and the DLS technique, and other measurement methods may be used.

### <DLS technique>

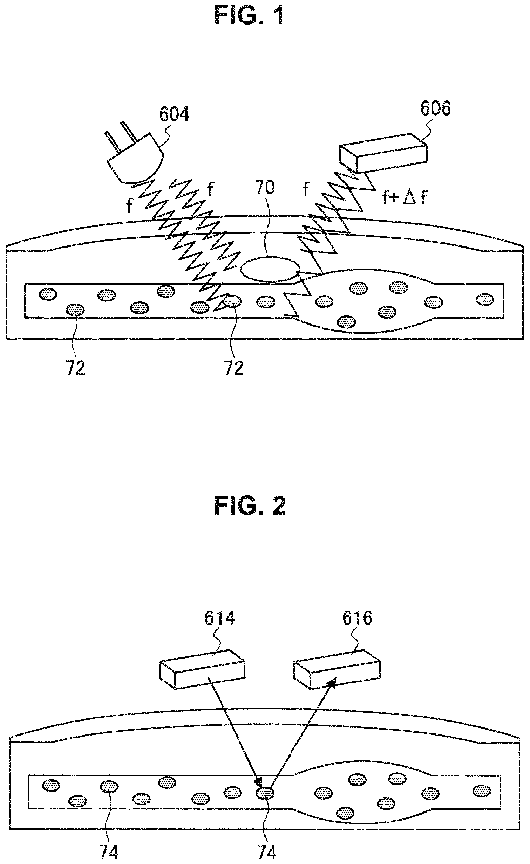

First, the DLS technique which is an example of the blood flow velocity measurement method applicable to an embodiment of the present disclosure will be described with reference to FIG. 1. FIG. 1 is an explanatory diagram for describing the DLS technique applied to an embodiment of the present disclosure.

The DLS technique is a method using a phenomenon in which, when a measurement region of a measured person is irradiated with laser light, the light is scattered by a scattering material (mainly red blood cells) moving in the blood of the measured person, and the scattered light causes interference light due to the Doppler effect. The interference light is received by a detector such as photodetector, and the blood flow velocity is calculated from a width of a Doppler shift frequency in the received interference light. Further, in the DLS technique, the blood flow velocity is calculated as an average tissue blood flow rate of a biological tissue within the measurement region, that is, a range that the irradiated laser light reaches.

Specifically, as illustrated in FIG. 1, in a case in which light of a frequency f with which the measurement region of the measured person is irradiated by an irradiating apparatus 604 is scattered by stationary tissue 70 which is stationary such as skin or subcutaneous tissue of the measured person, the scattered light maintains the frequency f. On the other hand, in a case in which the light of the frequency f with which the measurement region of the measured person is irradiated is scattered by a scattering material (for example, red blood cells) 72 moving through the blood vessels of the measured person, the scattered light undergoes the frequency shift in accordance with a moving velocity of the scattering material 72 due to the Doppler effect, and maintains a frequency f+Δf. Then, the scattered light of the frequency f scattered by the stationary tissue 70 and the scattered light of the frequency f+Δf which is scattered by the moving scattering material 72 and causes the Doppler shift interfere with each other, and thus a detector 606 can detect interference light having an optical beat (beat). Further, in general, the shift frequency Δf is much smaller than the frequency f of the irradiation light. The blood velocity is calculated by analyzing the interference light obtained by the detector 606 by performing, for example, a fast Fourier transform (FFT). Specifically, after the FFT is performed on the interference light, weighting is performed for each frequency, and a first moment is calculated by integrating with an appropriate frequency range. Further, the blood flow velocity (the blood flow rate per unit time) is calculated by integrating and normalizing a proportional constant to the calculated first moment. Further, in the DLS technique, since the blood flow velocity can be calculated if the frequency shift can be detected, it is possible to calculate the blood flow velocity even when the intensity of the interference light is weak.

Further, as a method of calculating the blood flow velocity from the interference light, in addition to the method of calculating using the FFT as described above, a method of calculating an auto-correlation function from the interference light and calculating the blood flow velocity using the calculated auto-correlation function can be used as well. The auto-correlation function is a measure illustrating how well a signal (interference light) at a certain time T and a signal (interference light) shifted by a time ΔT from the time T are matched and is expressed as a function of a shift time ΔT. The auto-correlation function obtained from the interference light by the blood flow has a form in which it attenuates as the shift time ΔT increases. Further, the tendency of the attenuation varies depending on the blood flow velocity, and in a case in which the blood flow velocity is slow, the auto-correlation function slowly attenuates as the shift time ΔT increases. On the other hand, in a case in which the blood flow velocity is fast, the auto-correlation function abruptly attenuates as the shift time ΔT increases. Therefore, the method using the auto-correlation function indirectly calculates the blood flow velocity (blood flow velocity) by referring to the attenuation tendency of the auto-correlation function.

Further, although all of the methods described above can be used as the method of calculating the blood flow velocity in embodiments of the present disclosure to be described below, in the following description, a case using the method of calculating the blood flow velocity using the auto-correlation function will be described as an example.

### <PPG technique>

Next, the PPG technique which is an example of the blood flow velocity measurement method applicable to an embodiment of the present disclosure will be described with reference to FIG. 2. FIG. 2 is an explanatory diagram for describing the PPG technique applied to an embodiment of the present disclosure.

The PPG technique is a measurement technique using a phenomenon in which an amount of light absorbed by a blood vessel varies with a volume change (pulse) of the blood vessel which occurs as the heart pumps out blood. Specifically, as illustrated in FIG. 2, in a case in which the measurement region of the measured person is irradiated with light (for example, green light) by an irradiating apparatus 614, the irradiation light is selectively absorbed mainly by red blood cells 74 in the blood of the measured person, and thus the absorption amount of the light is proportional to a blood amount (specifically, a tissue blood amount). In this regard, in the PPG technique, reflected light or transmitted light from the skin, the blood vessels, or the like of the measured person is detected by a detector 616 such as photodetector, and thus a change in the blood amount passing through for each pulse is obtained from the detection result, and the blood flow rate per unit time is calculated from the change in the blood amount.

Due to the difference in the measurement method, the DLS technique is known to be suitable for measuring the blood flow velocity in the blood vessels of the measurement region including not only the vicinity of the surface of the skin but also the deep blood vessels. On the other hand, the PPG technique is known to be suitable for measuring blood flow velocity in the capillary blood vessels located mainly near the surface of the skin of the measured person. Further, as described above, although both of the DLS technique and the PPG technique can be used in embodiments of the present disclosure, in the following description, the case using the DLS technique will be described as an example.

### <<2. Information processing system according to embodiment of present disclosure>>

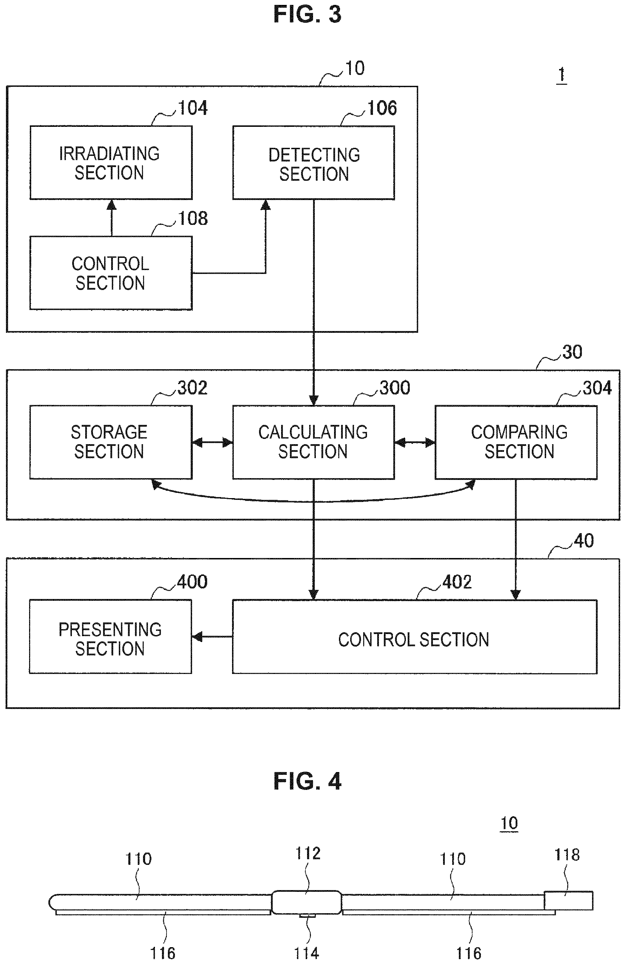

Next, an information processing system 1 according to an embodiment of the present disclosure will be described with reference to FIG. 3. FIG. 3 is a system diagram illustrating a configuration of the information processing system 1 according to an embodiment of the present disclosure. The information processing system 1 according to the present embodiment can measure the blood flow velocity of a measured person, perform a comparison using the measured blood flow velocity, and present information corresponding to a result of the comparison to a user. Further, by presenting the above information, the information processing system 1 can present the user with information encouraging behaviors (predetermined behaviors) that have an effect appropriate for the body of the measured person. As illustrated in FIG. 3, the information processing system 1 mainly includes a measuring module (measuring section) 10, a calculating apparatus 30, and an information presenting apparatus 40. Hereinafter, the measuring module 10, the calculating apparatus 30, and the information presenting apparatus (information presenting section) 40 included in the information processing system 1 will be described in order.

### <Measuring module 10>

The measuring module 10 is a module which is installed on the measurement region such as the skin of the measured person or the like in order to measure the blood flow velocity of the measured person. Further, the measuring module 10 can also measure the pulse of the measured person or the like by using the fact that the blood flow velocity periodically changes in accordance with the pulse. The measuring module 10 mainly includes an irradiating section 104, a detecting section 106, and a control section 108, as illustrated in FIG. 3. The respective components of the measuring module 10 will be described below.

### (Irradiating section 104)

The irradiating section 104 irradiates the measurement region of the measured person with irradiation light having a predetermined wavelength. A wavelength of the irradiation light irradiated by the irradiating section 104 can be appropriately selected. For example, in a case in which the DLS technique is used, a wavelength around 850 nm is selected, and a wavelength around 530 nm is selected in the PPG technique. As the irradiating section 104, in a case in which the DLS technique is used, a compact laser or the like can be used to irradiate with coherent light. Further, in a case in which the PPG technique is used, a green light emitting diode (LED) or the like can be used as the irradiating section 104. Further, one or more irradiating sections 104 are installed in the measuring module 10. Further, an irradiation timing, an irradiation period of time, an irradiation interval, an intensity, or the like of the irradiation light of the irradiating section 104 is controlled by the control section 108 to be described later.

### (Detecting section 106)

The detecting section 106 detects the interference light, the scattered light, or the transmitted light from the measurement region of the measured person in order to calculate the blood flow velocity. The detecting section 106 includes, for example, a photodiode (photo detector: PD), converts the intensity of the received light into an electric signal, and outputs the electric signal to the calculating apparatus 30 to be described later. Further, a charge coupled device (CCD) type sensor, a complementary metal oxide semiconductor (CMOS) type sensor, or the like can also be used as the detecting section 106. Further, one or more detecting sections 106 are installed in the measuring module 10. Further, the detecting section 106, that is, a timing or the like at which a detection result detected by the detecting section 106 is output as the electric signal (the detection result is read), is controlled by the control section 108 to be described later.

### (Control section 108)

The control section 108 is realized by, for example, a central processing unit (CPU), a read only memory (ROM), a random access memory (RAM), and the like. The control section 108 controls an irradiation pattern (an irradiation timing, an irradiation period of time, and an irradiation interval) of the irradiating section 104 on the basis of a predetermined synchronization signal or the like, controls a reading (sampling) timing of the detecting section 106, and controls measurement in the measuring module 10 in general. Further, the control section 108 may further include a storage section (not illustrated), and various kinds of programs, parameters, or the like for controlling the irradiating section 104 or the like may be stored in the storage section. Further, a timepiece mechanism (not illustrated) for detecting an accurate time in order to output the detection result of the detecting section 106 to the calculating apparatus 30 in association with the time may be installed in the control section 108.

Further, the measuring module 10 may include a communication section (not illustrated) or the like for communicating with the calculating apparatus 30 or the like in addition to the irradiating section 104, the detecting section 106, and the control section 108.

Further, for example, the measuring module 10 may have the form of a wearable apparatus which is used in a state in which it is worn on the body of a measured person. For example, the measuring module 10 may be an apparatus which has a shape such as a wristwatch type, a ring type, a wrist band type, an anklet type, a collar type, or the like and can be worn on a part of the body of the measured person such as a hand, an arm, a neck, or a leg. Further, the measuring module 10 may be an apparatus which has a pad shape such as an adhesive plaster type and can be worn on a part of the body of the measured person such as a hand, an arm, a neck, a leg or the like.

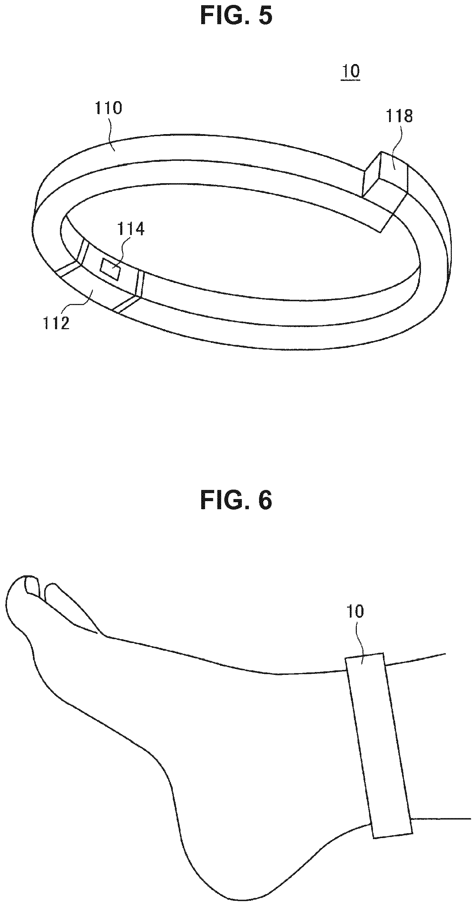

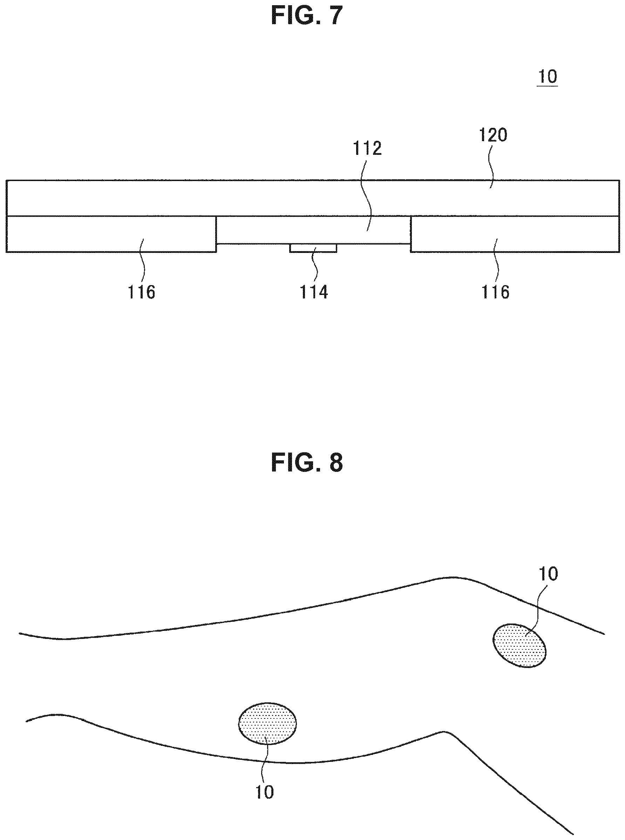

An example of a specific form of the measuring module 10 will be described below with reference to FIGS. 4 to 8. FIG. 4 is a diagram illustrating an example (anklet type) of a form of the measuring module 10 according to an embodiment of the present disclosure, and is a cross-sectional view taken in a long axis direction of a band-like measuring module 10. Further, FIG. 5 is an explanatory diagram for describing a form when the measuring module 10 illustrated in FIG. 4 is installed. FIG. 6 is an explanatory diagram for describing an installation example of the measuring module 10 illustrated in FIG. 4. FIG. 7 is a diagram illustrating another example (band plaster type) of the form of the measuring module 10 according to an embodiment of the present disclosure, that is, a cross-sectional view taken in a thickness direction of a pad-like measuring module 10. Further, FIG. 8 is an explanatory diagram for describing an installation example of the measuring module 10 illustrated in FIG. 7.

First, as an example of the form of the measuring module 10, the belt-like anklet type apparatus of FIG. 4 will be described. The measuring module 10 includes a belt-like band portion 110, a control unit 112, and a measuring unit 114 as illustrated in FIG. 4. The control unit 112 is a portion in which the control section 108 is installed. Further, in a case in which the measuring module 10 and the "calculating apparatus 30" to be described later are an integrated apparatus, respective components of the calculating apparatus 30 to be described later may be installed in the control unit 112. Further, the measuring unit 114 is a portion in which the irradiating section 104 and the detecting section 106 are installed.

The band portion 110 is a component which is wrapped around the ankle of a measured person and fixes the measuring module 10, has a ring-like form illustrated in FIG. 5 in accordance with the shape of the ankle, and is made of material such as a soft silicone gel or the like. In other words, since the band portion 110 can have a ring-shaped form along the ankle, the measuring module 10 can be wrapped around the ankle of the measured person and fixed as illustrated in FIG. 6.

Further, since accurate measurement is unable to be performed if the measuring module 10 moves during the measurement of the blood flow velocity, it is preferable that the measuring module 10 be fixed on the measurement region of the measured person. In this regard, an adhesive layer 116 attachable to the skin of the measured person is installed in a portion of the band portion 110 that comes in contact with the skin of the measured person. For example, the adhesive layer 116 is formed of a pressure sensitive adhesive (PSA) or the like such as a silicone pressure sensitive adhesive having a soft touch, and the adhesive layer 116 is affixed to the skin of the measured person to fix the measuring module 10. Further, since the adhesive layer 116 is soft, a good wearing feeling can be given to the measured person without placing a burden on the skin of the measured person. Further, by causing adhesion of the adhesive layer 116 to be in an appropriate state, the band portion 110 can be peeled from the skin without placing a burden on the skin of the measured person.

Further, it is preferable that, when the measuring module 10 has a ring-like form, a length of a circumference of the ring be freely adjustable so that various ankle thicknesses can be accepted. For example, in a case in which the measuring module 10 is loosely wrapped around the ankle, obviously, the measuring module 10 is unable to be fixed to the ankle, and the blood flow velocity is unable to be accurately measured. On the other hand, in a case in which the measuring module 10 is tightly wrapped around the ankle, since the blood flow is obstructed by the measuring module 10, the blood flow velocity of the blood flow in the obstructed state is measured, and the blood flow velocity indicating the physical state of the measured person is unable to be measured. Therefore, it is necessary to fit the measuring module 10 around the ankle of the measured person.

In this regard, a method of including magnetic metal powders in the adhesive layer 116 and installing a magnet 118 at the end of the band portion 110 can be considered. Since magnetic force acts between the magnetic metal powders of the adhesive layer 116 and the magnet 118, the magnet 118 can be fixed at an optimum position matching the thickness of the ankle of the measured person. Further, since the magnetic metal powders are included in the entire adhesive layer 116, it is easy to finely adjust the position of the magnet 118. Accordingly, the measuring module 10 can be worn and fixed in accordance with the thickness of the ankle of the measured person. Further, in the present embodiment, the magnetic metal powders may be included in the silicone gel forming the band portion 110 as well as the adhesive layer 116.

Next, as another example of the form of the measuring module 10, the pad-like adhesive bandage type apparatus of FIG. 7 will be described. The measuring module 10 has a pad portion 120, a control unit 112, and a measuring unit 114 as illustrated in FIG. 7. The pad portion 120 is a sheet which sticks to the skin of the measured person and fixes the measuring module 10, similarly to the band portion 110, and the pad portion 120 is made of a material such as soft silicone gel so that it can be deformed into a shape conforming to the surface of the skin of the measured person, similarly to the band portion 110. Further, an adhesive layer 116 is installed in a portion of the pad portion 120 that comes in contact with the skin of the measured person, similarly to the band portion 110. The adhesive layer 116 is affixed to the skin of the measured person and fixes the measuring module 10. As illustrated in FIG. 8, an adhesive plaster type measuring module 10 can be affixed to the skins of various parts of the body (legs in FIG. 8) of the measured person. Further, the control unit 112 and the measuring unit 114 are similar to the measuring module 10 in FIG. 4 described above, and thus description thereof is omitted here.

### <Calculating apparatus 30>

Returning to FIG. 3 again, the calculating apparatus 30 of the information processing system 1 according to the present embodiment will be described. The calculating apparatus 30 is an apparatus that calculates the blood flow velocity using the measurement result obtained by the measuring module 10 and compares the calculated blood flow velocity. As illustrated in FIG. 3, the calculating apparatus 30 mainly includes a calculating section 300, a storage section 302, and a comparing section 304. The respective components of the calculating apparatus 30 will be described below.

### (Calculating section 300)

The calculating section 300 is realized by, for example, a CPU, a ROM, a RAM, and the like. The calculating section 300 processes the measurement result obtained by the measuring module 10 and calculates the blood flow velocity. The calculated blood flow velocity can be output to the storage section 302 to be described later or output to the information presenting apparatus 40 to be described later. Further, a timepiece mechanism (not illustrated) that detects an accurate time in order to associate the blood flow velocity calculated by the calculating section 300 with a calculation time may be installed in the calculating section 300. For example, in a case in which the blood flow velocity is calculated, the calculating section 300 associates the calculated blood flow velocity with a time at which the measurement result is obtained from the measuring module 10 to calculate the blood flow velocity, and outputs an association result to the storage section 302 to be described later. Further, the calculating section 300 may analyze a waveform obtained from a temporal change in the blood flow velocity, generates consecutive information using the measurement result intermittently obtained by the measuring module 10, and calculates the blood flow velocity using the generated consecutive information.

### (Storage section 302)

The storage section 302 is realized by a RAM, a storage apparatus, or the like. The storage section 302 stores programs and various kinds of data used for the analysis process in the calculating section 300, programs and various kinds of data used in the comparing section 304 to be described later, the blood flow velocity calculated by the calculating section 300, and the like. Further, the storage section 302 may store various kinds of programs, parameters, data, or the like used for controlling the measuring module 10. Further, the storage section 302 may appropriately store various parameters which have to be stored when a certain process is performed, an interim result of the process, and the like in addition to such data. Then, the calculating section 300 or the like can freely access the storage section 302 and perform writing or reading.

### (Comparing section 304)

The comparing section 304 is realized by, for example, a CPU, a ROM, a RAM, and the like. The comparing section 304 compares the blood flow velocity calculated by the calculating section 300 with data stored in the storage section 302 (such as the blood flow velocity measured at a time different from that of the blood flow velocity). The comparing section 304 determines the blood flow state or the like of the measured person in accordance with a comparison result and controls the information presenting apparatus 40 to be described later. Further, the comparing section 304 can compare the waveform obtained from the temporal change in the blood flow velocity, count the number of times in a case in which a predetermined comparison result is obtained, and compare a count number with a predetermined numerical value.

Further, the calculating apparatus 30 may include a communication section (not illustrated) or the like for communicating with the measuring module 10 or the like in addition to the calculating section 300, the storage section 302, and the comparing section 304.

For example, the calculating apparatus 30 may be an information processing apparatus such as a smartphone, a tablet, or a personal computer (PC) owned by a user or another information apparatus connected with another apparatus (for example, such as a massage apparatus that performs massage on the measured person or the like). Further, the calculating apparatus 30 may be an information processing apparatus such as a server installed at a place away from the measured person.

Further, the calculating apparatus 30 may be an apparatus integrated with the measuring module 10 or may be an apparatus integrated with the information presenting apparatus 40 to be described later.

### <Information presenting apparatus 40>

Next, the information presenting apparatus 40 of the information processing system 1 according to the present embodiment will be described. The information presenting apparatus 40 presents the blood flow velocity calculated by the calculating apparatus 30 to the measured person or the user of the information processing system 1. Further, the information presenting apparatus 40 can present predetermined information for encouraging a predetermined behavior to the measured person in accordance with the comparison result (determination result) of the comparing section 304 using the blood flow velocity of the measured person. As illustrated in FIG. 3, the information presenting apparatus 40 mainly includes a presenting section 400 and a control section 402. The respective components of the information presenting apparatus 40 will be described below.

### (Presenting section 400)

The presenting section 400 is not particularly limited in a configuration thereof so long as information can be presented to the user. For example, the presenting section 400 may be a vibration generating apparatus with a small motor and is worn on the body of the measured person and presents predetermined information to the measured person by giving a vibration to the measured person. Further, the presenting section 400 may be a display apparatus such as a liquid crystal display (LCD) apparatus, an organic light emitting diode (OLED) apparatus, or the like. The presenting section 400 which is the display apparatus can display the blood flow velocity calculated by the calculating apparatus 30 for the user or display a predetermined message for the user in accordance with the calculated blood flow velocity. Further, the presenting section 400 which is the display apparatus may include a touch panel installed to be overlaid on the display apparatus. In this case, the presenting section 400 functions as a manipulating section that receives an input manipulation from the user, and outputs a signal corresponding to the received input manipulation to another functional section of the information presenting apparatus 40, the measuring module 10, or the calculating apparatus 30. Further, the presenting section 400 may be an audio output apparatus such as a speaker, or the like, and in this case, a predetermined voice message can be output to the user in accordance with the blood flow velocity calculated by the calculating apparatus 30.

### (Control section 402)

The control section 402 is realized by, for example, a CPU, a ROM, a RAM, and the like. The control section 108 controls the presenting section 400 on the basis of the output from the calculating apparatus 30 or the like, and controls an overall information presentation in the information presenting apparatus 40.

Further, the information presenting apparatus 40 may include a communication section (not illustrated) for communicating with the calculating apparatus 30 or the like.

The information presenting apparatus 40 may be realized by an information processing apparatus such as a smartphone, a tablet, a PC, or the like owned by the user which includes the vibration generating apparatus, the display apparatus, the audio output apparatus, or the like or may be an information processing apparatus connected with another apparatus (for example, a massage apparatus or the like).

Further, the information presenting apparatus 40 may be an apparatus integrated with the measuring module 10 or may be an apparatus integrated with the calculating apparatus 30.

Further, the information processing system 1 may include other apparatuses other than the measuring module 10, the calculating apparatus 30, and the information presenting apparatus 40. For example, the information processing system 1 may include a state detecting apparatus (not illustrated) that detects a posture, a state, or the like of the measured person, a massage apparatus that performs massage on the measured person, or the like.

### <<3. Information processing method according to first embodiment of present disclosure>>

Next, an information processing method according to a first embodiment of the present disclosure will be described. The information processing system 1 according to the present embodiment executes information processing to function as an economy class syndrome prevention apparatus.

It is known that, in a case in which a person is taking the same posture for a long time, for example, in a case in which a person moves with an airplane, an automobile, or the like or is setting to do a sedentary work for a long time or in a case in which a person has been lying down for a long time due to illness or the like and has not moved the legs, the person is likely to has an economy class syndrome. The economy class syndrome is an alias of deep vein thrombosis, pulmonary embolism occurs in a case in which, when thrombus which is a mass of blood is formed in the blood vessel, the thrombus causes swelling in a leg or an arm, and the thrombus reaches the lung. It is known that it is possible to prevent the onset of the economy class syndrome by performing an action of moving the legs or the like. Further, one of factors forming the thrombus causing the economy class syndrome is one in which, when the same posture has been taken for a long time, a calf muscle does not contract, a motion of pumping the blood out to the heart becomes weak, and the blood flow becomes slow.

In this regard, in the information processing system 1 according to the present embodiment, when the measured person is in a posture having a possibility of the onset of the economy class syndrome, for example, a seated posture, the blood flow velocity of the measured person is measured. Further, in the information processing system 1, in a case in which a decrease in the blood flow velocity of the measured person is detected, it is determined that there is a sign of the onset of the economy class syndrome, and the decrease in the blood flow velocity is presented to the measured person to encourage behaviors that prevent the economy class syndrome (encourage the measured person to do, for example, a preventive behavior of moving the legs). As a result, according to the information processing system 1 according to the present embodiment, the onset of the economy class syndrome can be prevented in advance.

### <Information processing system>

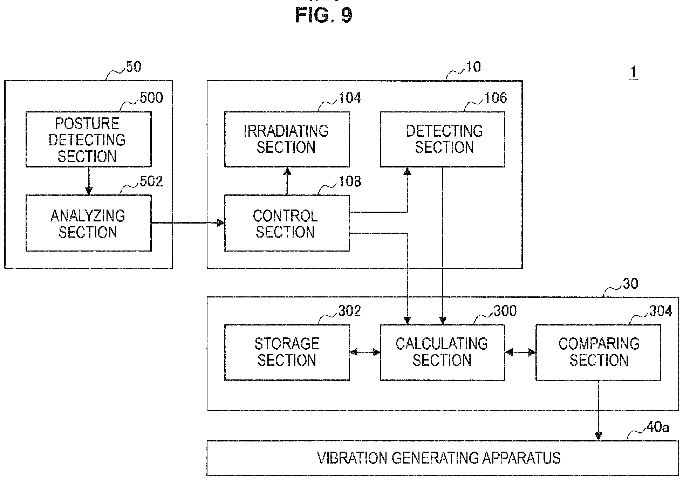

Hereinafter, the information processing system 1 according to the present embodiment will be described with reference to FIG. 9. FIG. 9 is a system diagram illustrating a configuration of the information processing system 1 according to the present embodiment. As illustrated in FIG. 9, the information processing system 1 includes a posture detecting apparatus 50 that detects a posture of the measured person in addition to the measuring module 10, the calculating apparatus 30, and a vibration generating apparatus 40a (an example of the information presenting apparatus 40).

Further, the information processing system 1 preferably has the following configuration in which wireless communication is not used so that the information processing system 1 can be used even when the measured person boards an airplane in which wireless communication is restricted. In other words, in the information processing system 1, it is preferable that the measuring module 10, the calculating apparatus 30, the vibration generating apparatus 40a, and the posture detecting apparatus 50 constitute an integrated apparatus. Further, the information processing system 1 is preferably a wearable apparatus which is used in a state in which it is worn on the body of the measured person in order to measure the blood flow velocity consecutively without placing burden on the measured person. In this regard, in the following description, for example, a case in which the information processing system 1 is an anklet type wearable apparatus as illustrated in FIG. 5 will be described. The anklet type wearable apparatus is worn on the ankle of the measured person and detects the presence or absence of the decrease in the blood flow velocity of the lower leg of the measured person on the basis of the change in the blood flow velocity near the ankle of the measured person.

In the present embodiment, the information presenting apparatus 40 is preferably compact to be incorporated into the wearable apparatus, and may be implemented by, for example, the vibration generating apparatus 40a which is worn on the body of the measured person and presents information to the measured person by giving the vibration. Further, a manipulating section (not illustrated) that receives a manipulation to start information processing to be described below or a manipulation to end the information processing from the measured person may be installed in any one of the measuring module 10, the calculating apparatus 30, the vibration generating apparatus 40a, and the posture detecting apparatus 50. In a case in which the manipulation is received, the manipulating section outputs a manipulation signal corresponding to the manipulation to the measuring module 10 or the like, so that the information processing is started or ended.

Specifically, as illustrated in FIG. 9, the information processing system 1 mainly includes the measuring module 10, the calculating apparatus 30, the vibration generating apparatus 40a, and the posture detecting apparatus 50. Further, in the following description, since the measuring module 10, the calculating apparatus 30, and the vibration generating apparatus 40a have been described above, only the posture detecting apparatus 50 will be described.

### (posture detecting apparatus 50)

The posture detecting apparatus 50 detects the posture of the measured person. As illustrated in FIG. 9, the posture detecting apparatus 50 includes a posture detecting section 500 and an analyzing section 502. Specifically, the posture detecting section 500 may be an acceleration sensor, a gyro sensor, or the like, and one or more posture detecting sections 500 are installed on predetermined parts of the body of the measured person and detect the motion of the measured person. For example, it is possible to detect the posture of the measured person by detecting a change in an acceleration, an angular velocity, or the like caused by an action of the measured person and analyzing a detection result indicating the detected change through the analyzing section 502. Further, the posture detecting section 500 may be an imaging device such as a camera or the like, and can detect the posture of the measured person by analyzing captured images of the measured person consecutively captured by the imaging device through the analyzing section 502. Further, the analyzing section 502 can also output an analysis result to the calculating apparatus 30 or the like. Further, the analysis by the analyzing section 502 may be performed in the calculating apparatus 30. Further, in the present embodiment, the configuration of the posture detecting apparatus 50 such as the posture detecting section 500 is not particularly limited, and any other configuration may be used as long as the apparatus can detect the posture of the measured person. Further, in a case in which the posture detecting apparatus 50 does not constitute an integrated apparatus with the measuring module 10, the calculating apparatus 30, and the vibration generating apparatus 40a as described above, the posture detecting apparatus 50 may include a communication section (not illustrated) for communicating with these apparatuses.

Further, the posture detecting apparatus 50 is not limited to an apparatus that detects the posture of the measured person and may be a state detecting apparatus (state detecting section) that detects information related to the physical state of the measured person. For example, the state detecting apparatus is a sensor for detecting biometric information of the measured person, and is worn directly on the body of the measured person and acquires information related to the physical state of the measured person such as a brain wave, respiration, sweating, myoelectric potential, or skin temperature.

### <Information processing method>

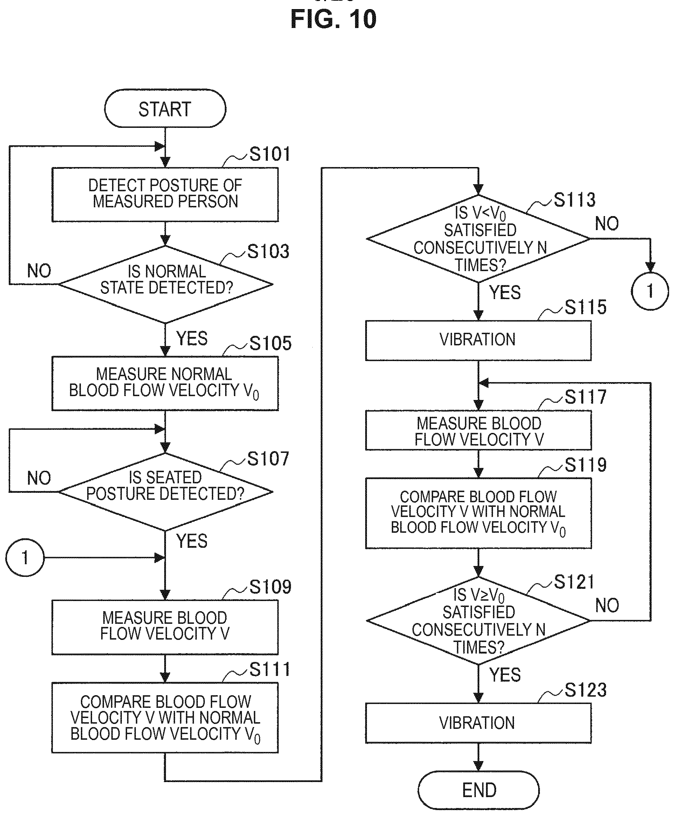

Next, an information processing method according to the present embodiment will be described with reference to FIG. 10. FIG. 10 is a flowchart illustrating an information processing method according to the present embodiment. First, if a general flow of the information processing method in the present embodiment is described, the measuring module 10 measures a normal blood flow velocity V₀ in a normal state before the measured person enters a predetermined state (enters a predetermined posture). Then, in a case in which the posture detecting apparatus 50 detects that the measured person enters a predetermined state such as a seated posture, the measuring module 10 measures the blood flow velocity V of the measured person in the predetermined state. Then, the calculating apparatus 30 compares the measured blood flow velocity V with the normal blood flow velocity V₀, and determines a blood flow state of the measured person. Further, in a case in which the decrease in the blood flow velocity indicating the sign of the onset of the economy class syndrome is detected with the comparison, it is determined that the measured person has the sign of the onset, and the vibration generating apparatus 40a presents predetermined information such as information indicating that the blood flow velocity of is decreasing to the measured person.

First of all, before the information processing according to the present embodiment is started, the wearable apparatus is worn on the ankle of the measured person as illustrated in FIG. 5. For example, before it becomes a situation in which the economy class syndrome is likely to occur such as before the measured person is scheduled to board an airplane is scheduled or starts a sedentary work, the wearable apparatus is worn on the measured person.

### (step S101)

In a case in which a manipulation signal corresponding to the manipulation to start the information processing from the measured person is detected, the posture detecting apparatus 50 starts detecting the posture of the measured person. In this case, the measuring module 10 may start measuring the blood flow velocity of the measured person.

### (Step S103)

The posture detecting apparatus 50 analyzes the detection result in step S101 and acquires information of the posture of the measured person. In a case in which the posture detecting apparatus 50 acquires information indicating that the measured person is in the posture in the normal state, the process proceeds to step S105. On the other hand, in a case in which the posture detecting apparatus 50 fails to acquire the information indicating that the measured person is in the posture of the normal state, the process returns to step S101. Further, the posture of the normal state is a posture of rest in a case in which the blood flow velocity of the measured person is a stable velocity. For example, the posture of the normal state may be a recumbent position which is a bedtime posture, a seated posture, or a state in which the same posture has not been taken for a long time, for example, about one or two hours. At this time, the posture detecting apparatus 50 may also determine whether the measured person is in the posture of the normal state with reference to the pulse of the measured person obtained by the measurement of the measuring module 10 as well.

### (Step S105)

The measuring module 10 measures the blood flow velocity of the measured person (for example, single blood flow velocity measurement is performed by performing sampling of interference light to be described later twice or more), and the calculating apparatus 30 calculates the blood flow velocity from the measurement result of the measuring module 10. In the following description, the blood flow velocity obtained in step S105 is referred to as a normal blood flow velocity V₀. Then, the calculating apparatus 30 stores the calculated normal blood flow velocity V₀. Further, the normal blood flow velocity V₀ may be an average value calculated using a plurality of measurement results obtained by measuring the blood flow velocity of the measured person twice or more. Further, separately, the normal blood flow velocity V₀ may be a blood flow velocity measured by the measuring module 10 when the measured person is in the normal state. In this case, the measured blood flow velocity is stored in the calculating apparatus 30 in advance as the normal blood flow velocity V₀, and execution of step S101 and step S103 can be omitted. Further, the normal blood flow velocity V₀ may be a blood flow velocity of the measured person which is measured after a predetermined period of time (for example, several minutes to several tens of minutes) elapses from a moment at which the measured person is detected to be in the seated posture in step S105 to be described later or from a time point at which the measured person is detected to be in the seated posture.

### (Step S107)

The posture detecting apparatus 50 consecutively acquires the information of the posture of the measured person. In a case in which the posture detecting apparatus 50 acquires information indicating that the measured person is in the seated posture, the process proceeds to step S109. On the other hand, in a case in which the posture detecting apparatus 50 fails to acquire the information indicating that the measured person is in the seated posture, step S107 is repeated. Much of the economy class syndromes are often caused when the seated posture is continuously taken for a long time. Therefore, in the present embodiment, the measurement of the blood flow velocity and the feedback of the information related to the blood flow velocity to the measured person are started when the measured person is detected to be in the seated posture.

### (Step S109)

The measuring module 10 measures an elapsed time since the measured person enters the seated posture, and in a case in which a predetermined period of time (for example, about several tens of minutes) elapses since it becomes the seated posture, the measurement of the blood flow velocity V of the measured person is performed (for example, single blood flow velocity measurement is performed by performing sampling of interference light to be described later twice or more).

### (Step S111)

The calculating apparatus 30 compares the blood flow velocity V obtained in step S109 with the normal blood flow velocity V₀ which has already been stored. In a case in which the blood flow velocity V is lower than the normal blood flow velocity V₀ (V<V₀), the calculating apparatus 30 increases the count number by 1 and stores it. Further, in a case in which the blood flow velocity V is higher than the normal blood flow velocity V₀ (V>V₀), the calculating apparatus 30 resets the count number back to zero and stores it.

### (Step S113)

The calculating apparatus 30 determines whether or not the count number counted in step S111 reaches a predetermined number N (the predetermined number N is a number that can be set in advance, and for example, an arbitrary natural number can be selected as the predetermined number N). In a case in which the calculating apparatus 30 determines that the count number reaches the predetermined number N, the process proceeds to step S115. On the other hand, in a case in which the calculating apparatus 30 determines that the count number does not reach the predetermined number N, the process returns to step S109.

Further, in a case in which the count number does not reach the predetermined number N in step SI13, steps S109 to S113 are repeated until the count number reaches the predetermined number N. Specifically, in the information processing according to the present embodiment, the measurement of the blood flow velocity V in step S109, the comparison of the blood flow velocity V in step S111, and the comparison of the count number in step S113 are repeated each time a predetermined period of time (for example, several tens of minutes) elapses since it becomes the seated posture.

In other words, in the information processing according to the present embodiment, in a case in which the blood flow velocity V is equal to or less than the normal blood flow velocity V₀ consecutively N times in a plurality of measurements of the blood flow velocity after the measured person enters the seated posture, it is determined that the measured person is in the state in which the economy class syndrome is likely to occur. Further, the determination method in step S113 is not limited to the above-described method, and any other determination method may be used. For example, the blood flow velocity of the measured person may be measured consecutively a predetermined number of times (for example, then times), and in a case in which the blood flow velocity V is lower than a value corresponding to a predetermined ratio (for example, 80%) of the normal blood flow velocity V₀ a predetermined number of times (for example, seven times) or more, it is determined that the measured person is in the state in which the economy class syndrome is likely to occur.

### (Step S115)

On the basis of the determination indicating that the measured person is in the state in which the economy class syndrome is likely to occur in step S113, the vibration generating apparatus 40a gives a vibration to the measured person and presents information indicating that the measured person is in the state in which the economy class syndrome is likely to occur. The vibration generating apparatus 40a encourages the measured person to do behaviors that prevent the economy class syndrome (for example, a preventive behavior of moving the legs) by presenting the information presentation based on the vibration. Further, the vibration generating apparatus 40a may include a mechanism for forcibly moving the legs of the measured person using a motor or the like, and in this case, the legs of the measured person may be forcibly moved as the vibration generating apparatus 40a is activated in step S115.

### (Step S117)

The measuring module 10 measures an elapsed time since the information presentation in step S115, and measures the blood flow velocity V of the measured person in a case in which a predetermined period of time (for example, several tens of minutes) elapses after the information presentation.

### (Step S119)

The calculating apparatus 30 compares the blood flow velocity V obtained in step S117 with the normal blood flow velocity V₀ which has already been stored. In a case in which the blood flow velocity V is higher than or equal to the normal blood flow velocity V₀ (V≥V₀), the calculating apparatus 30 increases the count number by 1 and stores it. Further, in a case in which the blood flow velocity V is lower than the normal blood flow velocity V₀ (V<V₀), the calculating apparatus 30 resets the count number back to zero and stores it.

### (Step S121)

The calculating apparatus 30 determines whether or not the count number counted in step S119 reaches a predetermined number N. In a case in which the calculating apparatus 30 determines that the count number reaches the predetermined number N, the process proceeds to step S123. On the other hand, in a case in which the calculating apparatus 30 determines that the count number does not reach the predetermined number N, the process returns to step S117. Further, the predetermined number N used in step S121 may be the same natural number as the predetermined number N used in step S113 or may be a natural number different from the predetermined number N used in step S113.

Further, in a case in which the count number does not reach the predetermined number N in step S121, steps S117 to S121 are repeated until the count number reaches the predetermined number N. Specifically, in the information processing according to the present embodiment, the measurement of the blood flow velocity V in step S117, the comparison of the blood flow velocity V in step S119, and the comparison of the count number in step S121 are repeated each time a predetermined period of time (for example, several tens of minutes) elapses since the information is presented.

In other words, in the information processing according to the present embodiment, when the blood flow velocity V higher or equal to the normal blood flow velocity V₀ is obtained N times in step S121, the reason is estimated to be because the measured person has done the preventive behavior encouraged by the information presentation in step S115. Further, similarly to step S113, the determination method in step S121 is not limited to the above-described method, and any other determination method may be used. For example, the blood flow velocity of the measured person may be measured consecutively a predetermined number of times (for example, then times), and in a case in which the blood flow velocity V is higher than or equal to a value corresponding to a predetermined ratio (for example, 80%) of the normal blood flow velocity V₀ a predetermined number of times (for example, seven times) or more, it is determined that the measured person gets out of the state in which the economy class syndrome is likely to occur.

### (Step S123)

On the basis of the determination in step S121, the vibration generating apparatus 40a gives a vibration to the measured person and presents information indicating that the measured person gets out of the state in which the economy class syndrome is likely to occur to the measured person. At this time, it is preferable that the vibration generating apparatus 40a generates a vibration having a pattern different from the vibration in step S115. Thereafter, in a case in which a manipulation signal corresponding to the manipulation to end the information processing from the measured person is detected, the information processing ends. Further, in the information processing according to the present embodiment, steps S101 to S123 may be repeated unless the manipulation signal is detected. Further, in the information processing, the information processing may be ended, for example, on the basis of an arbitrary index such as a change in the posture of the measured person, a change in the blood flow velocity, or a level of a battery (not illustrated) in the information processing system 1.

In the above description, the case in which the wearable apparatus in which the measuring module 10, the calculating apparatus 30, the information presenting apparatus 40, and the posture detecting apparatus 50 are integrated is used under the assumption that the measured person boards an airplane in which wireless communication is restricted has been described as an example. However, as described above, the onset of the economy class syndrome may occur not only in a case in which the person boards an airplane but also in a case in which the person is sitting in a car for a long time, for example, during driving or a sedentary work. In this case, since the use in an environment in which wireless communication is not restricted is considered, it is not limited to the example in which the wearable apparatus in which the measuring module 10, the calculating apparatus 30, the information presenting apparatus 40, and the posture detecting apparatus 50 are integrated is used. For example, a wearable apparatus in which the measuring module 10 and the posture detecting apparatus 50 are integrated may be used, and an information processing apparatus such as a smartphone in which the calculating apparatus 30 and the information presenting apparatus 40 are integrated may be used. In this case, for example, information indicating that the measured person is in the state in which the economy class syndrome is likely to occur may be presented on a display section (not illustrated) of the information processing apparatus. Further, the wearable apparatus is not limited to the ankle type illustrated in FIG. 5 but may be, for example, a pad-like adhesive plaster type wearable apparatus illustrated in FIG. 8.

Further, as described above, the onset of the economy class syndrome may occur not only in a case in which the person is sitting for a long time but also in a case in which the person is in a fixed posture (the same posture) for a long time, for example, while sleeping for a long time. Therefore, in the present embodiment, the posture detecting apparatus 50 is not limited to detecting that the measured person is in the seated posture and may detect that the measured person is in the fixed posture for a long time. Further, in a case in which the wearable apparatus has a state detecting apparatus (not illustrated) that detects biometric information related to the physical state of the measured person, the measurement of the blood flow velocity and the feedback of the information related to the blood flow velocity to the measured person may be started when the state detecting apparatus detects predetermined biometric information.

As described above, in the present embodiment, the measurement of the blood flow velocity of the measured person is performed in a case in which the measured person is in a posture in which the onset of the economy class syndrome is likely to occur. Further, in a case in which the decrease in the blood flow velocity of the measured person is detected, it is determined that there is a sign of the onset of the economy class syndrome, and the information indicating the decrease in the blood flow velocity is presented to the measured person to encourage the measured person to do the preventive behavior for preventing the economy class syndrome. As a result, according to the present embodiment, it is possible to prevent the onset of economy class syndrome in advance. In other words, according to the present embodiment, since the blood flow velocity measurement function provided by the wearable apparatus being worn is used, it is possible to encourage the behavior that has an effect appropriate to the body of the measured person depending on the physical condition of the measured person even at a stage in which there is no subjective symptom in the measured person. Further, in the present embodiment, the information indicating that the blood flow velocity is decreasing and the information indicating that the blood flow velocity is increasing since the preventive behavior has been done are presented to the measured person. According to the present embodiment, as such information is presented, it is possible to cause the preventive behavior of the measured person, to inform the measured person of the effect of the preventive behavior, to increase a sense of trust in the preventive function realized by the present embodiment.

### <<4. Information processing method according to second embodiment of present disclosure>>

Next, an information processing method according to a second embodiment of the present disclosure will be described. The information processing system 1 according to the present embodiment implements information processing to function as an auxiliary apparatus for appropriately installing a pressurizer in pressurization training.

The pressurization training is a training technique in which training is performed in a state in which a base of an arm or a leg is pressurized by a dedicated belt-like pressurizer, and the blood flow is properly limited. In the pressurization training, since the blood flow is moderately inhibited during the training, even relatively low load training can cause a large amount of lactic acid which is a fatigue material to be generated as in high-load training, and the lactic acid is accumulated in a muscle. As the large amount of lactic acid is accumulated in the muscle, a large amount of growth hormone is secreted, body fat can be decomposed, and the muscle can be formed. In other words, the pressurization training is a training technique which can obtain effects similar to those at the time of high-load training even through relatively low load training since the pressurization is performed. In this regard, in the pressurization training, it is required to properly limit the blood flow in order to obtain appropriate training effects. For example, in a case in which the blood flow is not restricted, desired training effects are unable to be obtained, and on the other hand, in a case in which the blood flow is excessively restricted, the blood does not flow in the muscles, resulting in oxygen shortage.

In this regard, in the present embodiment, the blood flow velocity before the pressurization is performed by the pressurizer is compared with the blood flow velocity after the pressurization, a comparison result is presented to the user, and guidance is given so that the pressurizer can be installed so that the blood flow can be appropriately restricted.

Further, in the following description, a "user" is a user who uses the information processing system 1 according to the present embodiment and includes, for example, a trainer who gives a training instruction to the measured person in addition to the measured person wearing the pressurizer.

### <Information processing system>

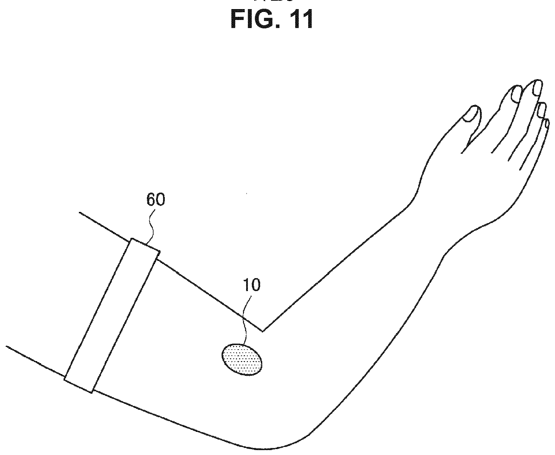

An information processing system 1 according to the present embodiment will be described below with reference to FIGS. 3, 8, and 11. FIG. 11 is an explanatory diagram for describing an installation example of the measuring module 10 according to the present embodiment. The information processing system 1 according to the present embodiment has the configuration illustrated in FIG. 3, and in the present embodiment, the presenting section 400 of the information presenting apparatus 40 is, for example, a display apparatus such as a liquid crystal display apparatus. The presenting section 400 displays the blood flow velocity calculated by the calculating apparatus 30 for the user or displays a predetermined message for the user in accordance with the calculated blood flow velocity. Further, the presenting section 400 which is a display apparatus may include a touch panel overlaid on the display apparatus, and in this case, the presenting section 400 can also function as a manipulating section that receives an input manipulation from the user.

Further, the measuring module 10 of the information processing system 1 according to the present embodiment preferably has, for example, the form of the adhesive plaster type wearable apparatus illustrated in FIG. 8. For example, in a case in which a pressurizer 60 is installed on the arm of the measured person as illustrated in FIG. 11, the measuring module 10 is installed in the vicinity of the pressurizer 60 in the arm of the measured person. Specifically, the measuring module 10 is installed in the vicinity of the pressurizer 60 at a position farther from the heart of the measured person than the pressurizer 60.

Further, in the present embodiment, the calculating apparatus 30 and the information presenting apparatus 40 may constitute an integrated apparatus, and may constitute, specifically, an information processing apparatus such as a smartphone, a tablet, a PC, or the like owned by the user. Further, the calculating apparatus 30 and the information presenting apparatus 40 may include a communication section (not illustrated) for communicating with the measuring module 10.

### <Information processing method>

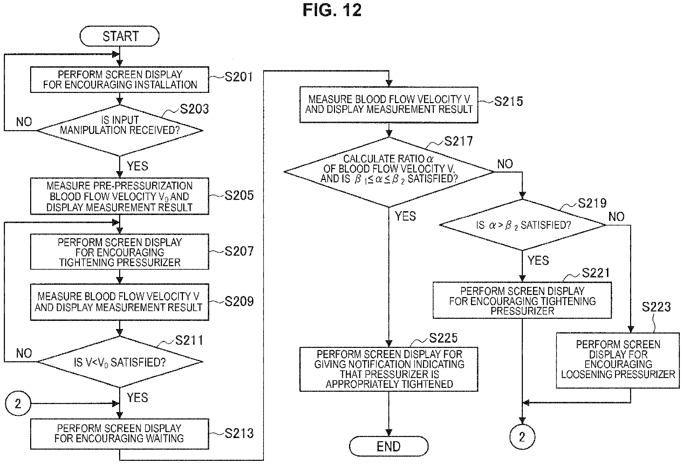

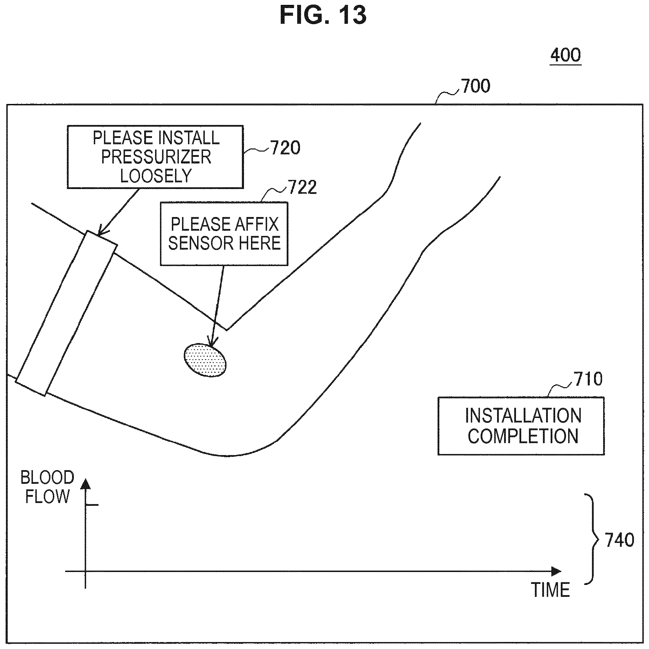

Next, an information processing method according to the present embodiment will be described with reference to FIGS. 12 to 17. FIG. 12 is a flowchart illustrating the information processing method according to the present embodiment. FIGS. 13 to 17 are explanatory diagrams for describing examples of a display screen according to the present embodiment. First, if a general flow of the information processing method in the present embodiment is described, the measuring module 10 measures a pre-pressurization blood flow velocity V₀ of the measured person before the pressurization by the pressurizer 60. Then, after the measured person is pressurized by the pressurizer 60, the measuring module 10 measures a post-pressurization blood flow velocity V of the measured person. Further, the calculating apparatus 30 compares the post-pressurization blood flow velocity V with the pre-pressurization blood flow velocity V₀, and in a case in which a comparison result is not a predetermined value, the information presenting apparatus 40 presents information for encouraging the user to tighten or loosen the pressurizer 60.

### (Step S201)

In a case in which the manipulation signal corresponding to the manipulation to start information processing from the user is detected, the information presenting apparatus 40 presents a screen 700 for encouraging the user to install the pressurizer 60 and the measuring module 10. Specifically, in a case in which the pressurizer 60 is installed on the arm of the measured person, the screen 700 includes a message 720 for loosely installing the pressurizer 60 on the arm of the measured person and a message 722 indicating a position at which the measuring module 10 is installed as illustrated in FIG. 13. Further, the screen 700 may include other displays besides the messages 720 and 722, and for example, as illustrated in FIG. 13, the screen 700 may include a graph 740 indicating a relation between an elapsed time after the measurement of the blood flow velocity is started and the blood flow velocity. Further, the screen 700 includes a button 710 for the user to give a notification indicating that the installation of the pressurizer 60 and the measuring module 10 is completed to the information presenting apparatus 40 (a rectangular manipulation button with characters of installation completion in FIG. 13).

The user installs the pressurizer 60 on a part of the body such as the arm of the measured person as illustrated in FIG. 11 in accordance with the guidance of the message 720. At this time, the user installs the pressurizer 60 on the body of the measured person so that it becomes a state that the body of the measured person is not pressurized by the pressurizer 60. Further, the user installs the measuring module 10 on a part of the body of the measured person corresponding to the position indicated by the message 722.

### (Step S203)

In a case in which the information presenting apparatus 40 receives the input manipulation for the user to give a notification indicating that the installation of the pressurizer 60 and the measuring module 10 is completed as a result of manipulating the button 710 by the user, the process proceeds to step S205. On the other hand, in a case in which the user does not manipulate the button 710 although a predetermined period of time (for example, several minutes) elapses, and the information presenting apparatus 40 does not receive the input manipulation for the user to give a notification indicating that the installation of the pressurizer 60 and the measuring module 10 is completed, the process returns to step S201.

### (Step S205)

The measuring module 10 measures the blood flow velocity V of the measured person, and the information presenting apparatus 40 displays the result of the measured blood flow velocity V as the graph 740. Further, the measuring module 10 measures the elapsed time from the measurement start, and measures the blood flow velocity V of the measured person each time a predetermined period of time (for example, several seconds) elapses from the measurement start. Further, the information presenting apparatus 40 consecutively displays the result of the measured blood flow velocity V. In a case in which the blood flow velocity V included within a predetermined range is measured a predetermined number of times, it is determined that a stable blood flow velocity is measured, and the calculating apparatus 30 calculates an average using a plurality of blood flow velocities V and regards the obtained average value as the pre-pressurization blood flow velocity V₀. The calculating apparatus 30 stores the calculated pre-pressurization blood flow velocity V₀.

### (Step S207)

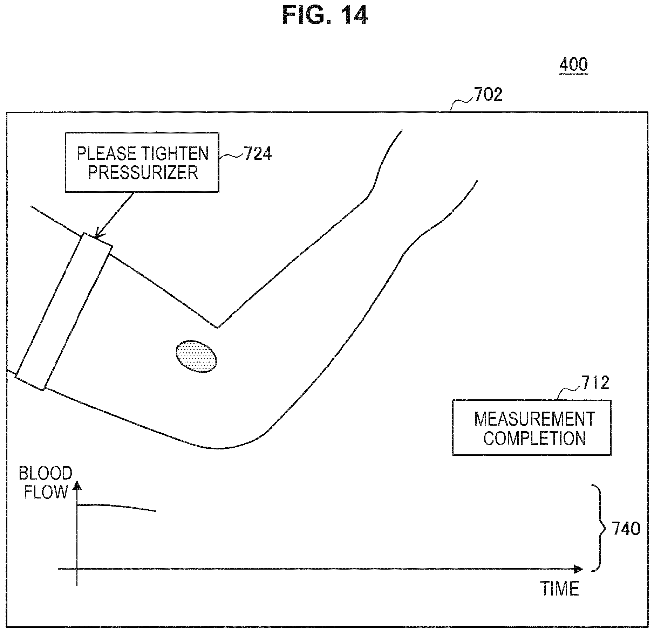

The information presenting apparatus 40 displays a screen 702 for encouraging the user to tighten the pressurizer 60. Specifically, the screen 702 includes a message 724 for encouraging the user to tighten the pressurizer 60 and a graph 740 illustrating a change in the measured blood flow velocity with respect to the elapsed time from the measurement start as illustrated in FIG. 14. The user is guided by the message 724, tightens pressurizer 60, and pressurizes the body of measured person. Further, in addition to the message 724 and the graph 740, the screen 702 may include another display such as an end button 712 for the user to give a notification indicating that the information processing is ended to the information presenting apparatus 40.

### (Step S209)

The measuring module 10 measures the blood flow velocity V of the measured person, and the information presenting apparatus 40 displays the result of the measured blood flow velocity V as the graph 740.

### (Step S211)

The calculating apparatus 30 compares the blood flow velocity V acquired in step S209 with the pre-pressurization blood flow velocity V₀ which is already stored. In a case in which the blood flow velocity V is determined to be lower than the pressurization blood flow velocity V₀ (V<V₀), the calculating apparatus 30 determines that the pressurizer 60 pressurizes the blood flow and so the blood flow is obstructed, the process proceeds to step S213. On the other hand, in a case in which the blood flow velocity V is determined to be equal to or higher than the pressurization blood flow velocity V₀ (V≥V₀), the calculating apparatus 30 determines that the pressurizer 60 is not being pressurized, and the process returns to step S207.

### (Step S213)

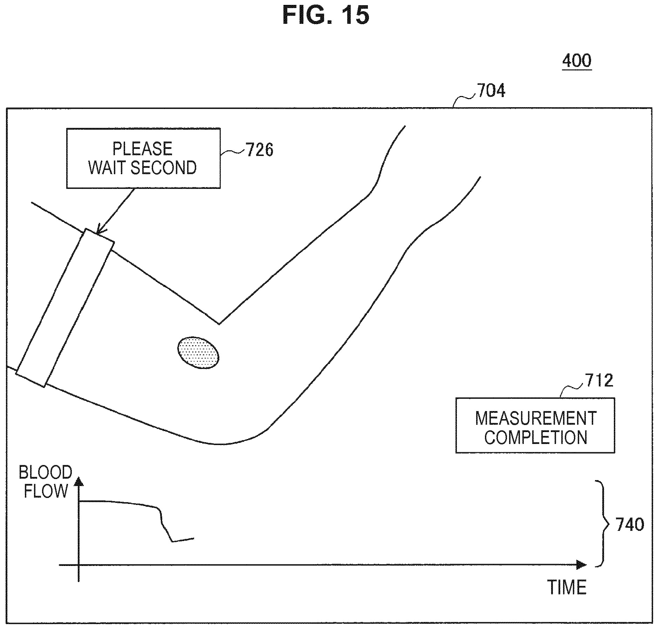

The presenting section 400 displays a screen 704 for encouraging the user to wait. Specifically, as illustrated in FIG. 15, the screen 704 includes a message 726 for encouraging the user to wait in order to measure the blood flow velocity in the stable state.

### (Step S215)

The measuring module 10 measures the blood flow velocity V of the measured person, and the information presenting apparatus 40 displays the result of the measured blood flow velocity V as the graph 740. Further, the measuring module 10 measures the elapsed time since the screen 704 is displayed in step S213, and measures the blood flow velocity V of the measured person each time a predetermined period of time (for example, several seconds) elapses from the display start. Further, the information presenting apparatus 40 consecutively displays the result of the measured blood flow velocity V. In a case in which the blood flow velocity V included within a predetermined range is measured a predetermined number of times, it is determined that a stable blood flow velocity is measured, and the calculating apparatus 30 calculates an average using a plurality of blood flow velocities V and regards the obtained average value as the pre-pressurization blood flow velocity V₀. The calculating apparatus 30 stores the calculated post-pressurization blood flow velocity V₁.

### (Step S217)

The calculating apparatus 30 compares a post-pressurization blood flow velocity V₁ obtained in step S207 with the pre-pressurization blood flow velocity V₀ which is already stored. Specifically, the calculating apparatus 30 calculates a ratio α (=V₁/V₀) of the post-pressurization blood flow velocity V₁ to the pre-pressurization blood flow velocity V₀, and determines whether or not the calculated α falls within a range of predetermined values β₁ to β₂ (β₁≦α≦ β₂). The range of the predetermined values β₁ to β₂ is a range having a predetermined width (Δβ) in which a ratio β₀ (=V₂/V₀) of the post-pressurization blood flow velocity V₂ obtained when properly pressurized to the pre-pressurization blood flow velocity V₀ is set as a center value. In other words, β₁ is a value obtained by subtracting half of the width Δβ from β₀ (β₁=β₀-Δβ/2), and β₂ is a value obtained by adding half of the width Δβ from β₀ (β₂=β₀+Δβ/2). In a case in which the ratio α is determined to fall within the range of the predetermined values β₁ to β₂, the calculating apparatus 30 determines that the pressurization is appropriately performed, and the process proceeds to step S225. On the other hand, in a case in which the ratio α is determined not to fall within the range of the predetermined values β₁ to β₂, the calculating apparatus 30 determines that the pressurization is not appropriately performed, and the process proceeds to step S219.

### (Step S219)

The calculating apparatus 30 compares the ratio α calculated in step S217 with β₂ used in step S217. In a case in which the ratio α is determined to be higher than β₂ (α>β₂), the calculating apparatus 30 determines that the training effects are unable to be obtained unless further pressurization is performed by the pressurizer 60, and the process proceeds to step S221. On the other hand, in a case in which the ratio α is determined not to fall within the range of the predetermined values β₁ to β₂ in step S217, and the ratio α is determined to be lower than the β₂ in step S219 (α<β₂), the calculating apparatus 30 determines that excessive pressurization is being performed by the pressurizer 60, and the process proceeds to step S223.

Further, in steps S217 and S209, the ratio α of the post-compression blood flow velocity V to the pre-pressurization blood flow velocity V₀ is calculated, and it is determined whether or not the pressurization is appropriate using the ratio α. However, the determination method in the present embodiment is not limited to the above-described determination method, and for example, a difference ΔV between the post-compression blood flow velocity V₁ and the pre-compression blood flow velocity V₀ may be calculated, and it may be determined whether or not the pressurization is appropriate using the calculated difference ΔV.

### (Step S221)

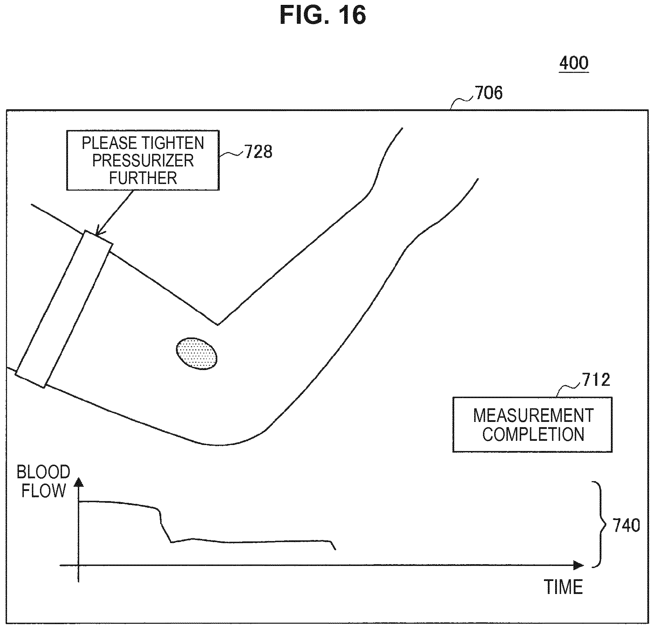

The information presenting apparatus 40 displays a screen 706 for encouraging the user to further tighten the pressurizer 60. Specifically, the screen 706 includes a message 728 for encouraging to tighten the pressurizer 60 and a graph 740 illustrating a change in the measured blood flow velocity with respect to the elapsed time from the measurement start as illustrated in FIG. 16. The user is guided by the message 728, tightens the pressurizer 60, and adjusts the pressure on the body of the measured person.

### (Step S223)

The information presenting apparatus 40 displays a screen (not illustrated) for encouraging the user to loosen the pressurizer 60. Specifically, similarly to the above screen 706, the screen includes a message for encouraging to loosen the pressurizer 60 and a graph 740 illustrating a change in the measured blood flow velocity with respect to the elapsed time from the measurement start. The user is guided by the message, loosens the pressurizer 60, and adjusts the pressure on the body of the measured person.

### (Step S225)

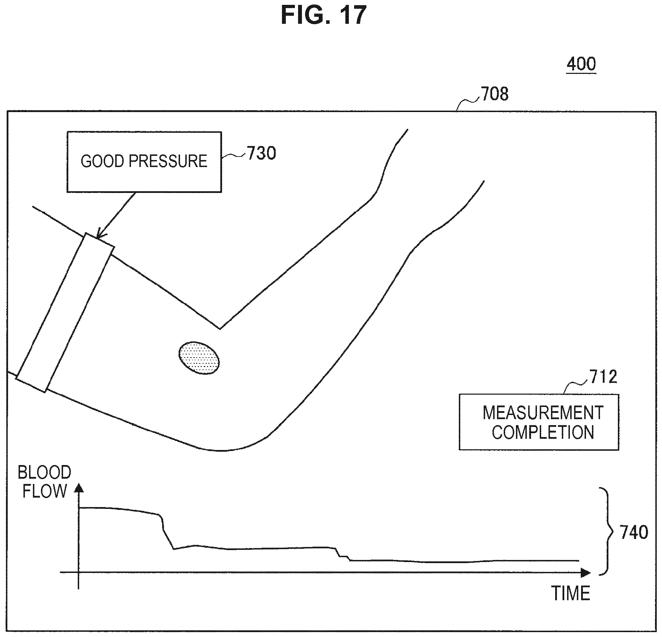

The information presenting apparatus 40 displays a screen 708 indicating that the pressurizer 60 is properly installed on the user. Specifically, as illustrated in FIG. 17, the screen 708 includes a message 730 indicating that the pressurizer 60 is properly installed and a graph 740 illustrating a change in the measured blood flow velocity with respect to the elapsed time from the measurement start. Further, the graph 740 illustrated in FIG. 17 indicates a history of the change in the blood flow velocity obtained when it becomes a state in which the pressurizer 60 properly pressurizes the body of the measured person as a result of further tightening the pressurizer 60 as the pressurizer 60 is urged to be further tightened one time. Thereafter, as illustrated in FIG. 12, the information processing may be ended, or in a case in which it is desired to see the change in the blood flow velocity during the pressure training, the measurement of the blood flow velocity may be continuously performed. In the latter case, by checking the change in the blood flow velocity according to the progress of the training, it is possible to more appropriately adjust the load of the training and adjust the pressurization by the pressurizer 60 in accordance with the physical condition of the measured person even during the training.

Further, in the present embodiment, the measuring module 10 is not limited to the adhesive plaster type but may have another form such as a bracelet type depending on an installation position of the pressurizer 60.

As described above, in the present embodiment, the pre-pressurization blood flow velocity V₀ before the pressurization by the pressurizer 60 is compared with the post-pressurization blood flow velocity V₁ after the pressurization, and a comparison result is presented to the user. Therefore, according to the present embodiment, since the comparison result is presented to the user, it is possible to induce the pressurizer 60 to enter the pressurizing state so that the blood flow can be appropriately restricted. In other words, according to the present embodiment, it is possible to appropriately install the pressurizer to give an effect appropriate to the body of the measured person depending on the physical condition of the measured person by using the blood flow velocity measurement function by the wearable apparatus being worn. Further, in the present embodiment, the blood flow velocity measurement results indicating that the blood flow velocity has decreased or increased by the pressurizer 60 are sequentially displayed, and thus it is possible to give convincing guidance can be given to the user.

### <<5. Information processing method according to third embodiment of present disclosure>>

Next, an information processing method according to a third embodiment of the present disclosure will be described. The information processing system 1 according to the present embodiment performs information processing to function as an auxiliary apparatus for properly performing massage while confirming effects of the massage.

There are cases in which it is desired to confirm effects of treatment such as a part of the body which has to be treated or an exercise to be performed and a part of the body in which the blood flow is improved (the blood flow velocity is increased) by the treatment or the exercise when treatment, an exercise, stretching, or the like of improving the blood flow such as the massage is performed. For example, effects of treatment such as the massage differ depending on a subject, and specifically, even in a case in which the same part of the body is treated, there are a subject for which it is effective and a subject for which it is not effective. Further, even in a case in which the same part of the body is treated, a part in which an effect such as improvement in the blood flow is shown may differ depending on a subject as well. In this regard, in the present embodiment, it is possible to review a treatment method or the like suitable for each subject by checking the blood flow velocity of the subject during the treatment or the like.

Further, in the following description, a "user" is a user who uses the information processing system 1 according to the present embodiment, and includes, for example, a practitioner or the like who performs treatment or the like on the subject in addition to the subject to be treated (the subject is also the measured person in the present embodiment). Further, in the following description, the subjects to be treated is also a blood flow velocity measurement target and thus referred to as a measured person.

### <Information processing system>

An information processing system 1 according to the present embodiment will be described below with reference to FIGS. 3 and 8. The information processing system 1 according to the present embodiment has the configuration illustrated in FIG. 3, and in the present embodiment, the presenting section 400 of the information presenting apparatus 40 is, for example, a display apparatus. The presenting section 400 can display the blood flow velocity for the user and display a predetermined message for the user in accordance with the blood flow velocity. Further, the presenting section 400 which is the display apparatus also includes a touch panel overlaid on the display apparatus and can function as a manipulating section that receives an input manipulation from the user.

Further, the measuring module 10 of the information processing system 1 according to the present embodiment preferably has, for example, the form of the adhesive plaster type wearable apparatus illustrated in FIG. 8. The adhesive plaster type wearable apparatus can be affixed and fixed to substantially all parts of the body of the measured person unless it is a part to be treated. Specifically, in the present embodiment, the measuring module 10 is affixed to a part in which it is desired to confirm the blood flow improvement in the body of the measured person (for example, near a muscle having a corium), and treatment such as the massage is performed on the part of the body away from the measuring module 10. At this time, the user performs the treatment while keeping a constant fixed state of the measuring module 10, and the measuring module 10 measures the change in the blood flow velocity of each part during the treatment if necessary. Therefore, since it is possible to confirm the blood flow velocity of the part of the body of the measured person during the treatment, it is possible to review an effective treatment suitable for the measured person.

Further, in the present embodiment, the calculating apparatus 30 and the information presenting apparatus 40 may constitute an integrated apparatus, and may constitute, specifically, an information processing apparatus such as a tablet or an information processing apparatus connected with a massage apparatus or the like that performs treatment on the measured person. Further, the calculating apparatus 30 and the information presenting apparatus 40 may have a communication section (not illustrated) for communicating with the measuring module 10.

### <Information processing method>

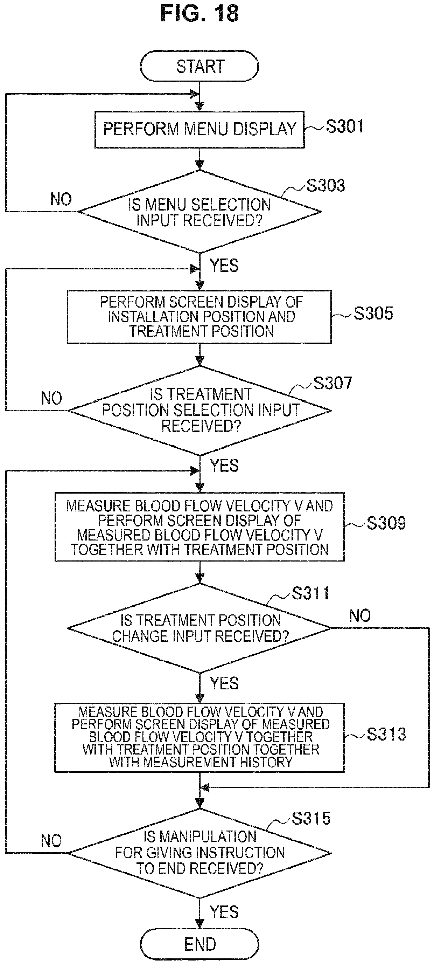

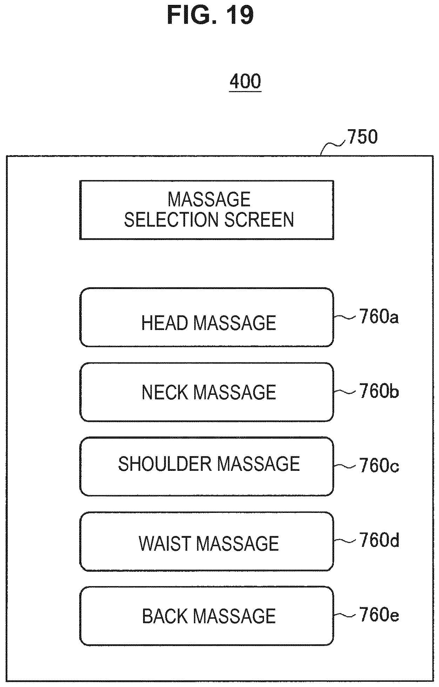

Next, an information processing method according to the present embodiment will be described with reference to FIGS. 18 to 23. FIG. 18 is a flowchart illustrating the information processing method according to the present embodiment. FIGS. 19 to 23 are explanatory diagrams for describing examples of a display screen according to the present embodiment. First, if a general flow of the information processing method in the present embodiment will be described, the information presenting apparatus 40 presents a position at which the measuring module 10 is installed and a position at which the treatment is performed to the user. Then, in accordance with the presentation of the information presenting apparatus 40, the user installs the measuring module 10 on the measured person and starts the treatment (massage) on each indicated part. During the treatment, the measuring module 10 measures the blood flow velocity of the measured person, and the information presenting apparatus 40 presents the measured blood flow velocity. Therefore, the user can confirm a part which has to be treated to increase the effect of the blood flow improvement during the treatment, and thus it is possible to review the treatment technique or the like suitable for the measured person.

### (Step S301)

In a case in which the manipulation signal corresponding to the manipulation to start the information processing from the user is detected, the information presenting apparatus 40 presents a menu screen 750 for selecting, for example, a massage menu to the user. Specifically, as illustrated in FIG. 19, the screen 750 includes a plurality of menu buttons 760a to 760e for selecting the massage menu. Each menu button 760 is a button for inputting a manipulation for selecting a part in which the user wishes to improve the blood flow, and for example, in a case in which it is desired to improve the blood flow of the muscles around the shoulder of the measured person, the user selects the button 760c indicating "shoulder massage." Further, in the following, a case in which the user selects the massage menu for improving the blood flow of the muscles around the shoulder will be described as an example. Further, the screen 750 may include other displays or the like in addition to a plurality of menu buttons 760a to 760e.

### (Step S303)

In a case in which the information presenting apparatus 40 receives the input manipulation for the user to select the massage menu (the massage menu for improving the blood flow of the muscles around the shoulder) as the user performs the manipulation on the button 760 (button 730c), the process proceeds to step S305. On the other hand, in a case in which the user does not perform the manipulation although a predetermined period of time (for example, several minutes) elapses, and the information presenting apparatus 40 did not receive the input manipulation for the user to select the massage menu, the process returns to step S301.

### (Step S305)

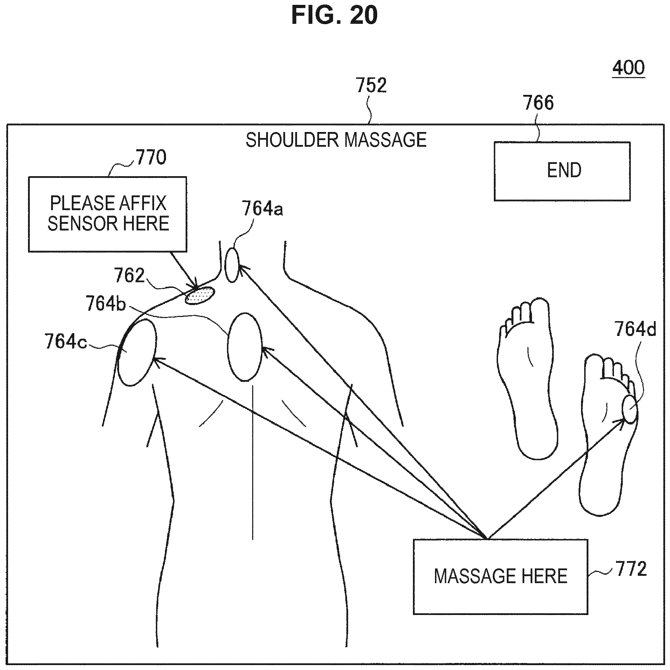

The information presenting apparatus 40 displays a screen 752 indicating an installation position at which the measuring module 10 is installed and a treatment position at which the treatment is performed for the user. Specifically, in a case in which the user selects the massage menu to improve the blood flow of the muscles around the shoulder, the screen 752 includes a message 770 indicating that the measuring module 10 is installed on the shoulder of the measured person as illustrated in FIG. 20. Further, the screen 752 includes a mark 762 indicating the position at which the measuring module 10 is installed on a schematic diagram of a human body. Further, the screen 752 also includes a message 772 indicating a treatment position and marks 764a to 764d indicating treatment positions on the schematic diagram of the human body. Further, the marks 764a to 764e also function as a manipulating unit for the user to selectively input the treatment positions. As the user performs the manipulation on the marks 764a to 764d, it is possible to notify the information presenting apparatus 40 of the treatment position at which the user is desired to perform the treatment. Further, in addition to the messages 770 and 772 and the marks 762 and 764, the screen 752 may include other displays such as an end button 766 for notifying the information presenting apparatus 40 of an instruction indicating that the user ends the information processing.

The user installs the measuring module 10 at the installation position (for example, the shoulder) of the measured person in accordance with the guidance by the message 770 and the mark 762. Further, the user performs the manipulation on the mark 764 corresponding to the treatment position at which the treatment is desired to be performed from among the marks 764a to 764d indicating the treatment positions. In the following description, it is assumed that the user selects the treatment position indicated by the mark 764a.

### (Step S307)

In a case in which the information presenting apparatus 40 receives the input manipulation for the user to select the treatment position as the user performs the manipulation on the mark 764, the process proceeds to step S309. On the other hand, in a case in which the user does not manipulate the mark 764 although a predetermined period of time (for example, several minutes) elapses, and the information presenting apparatus 40 does not receive the input manipulation for the user to select the treatment position, the process returns to step S305.

### (Step S309)

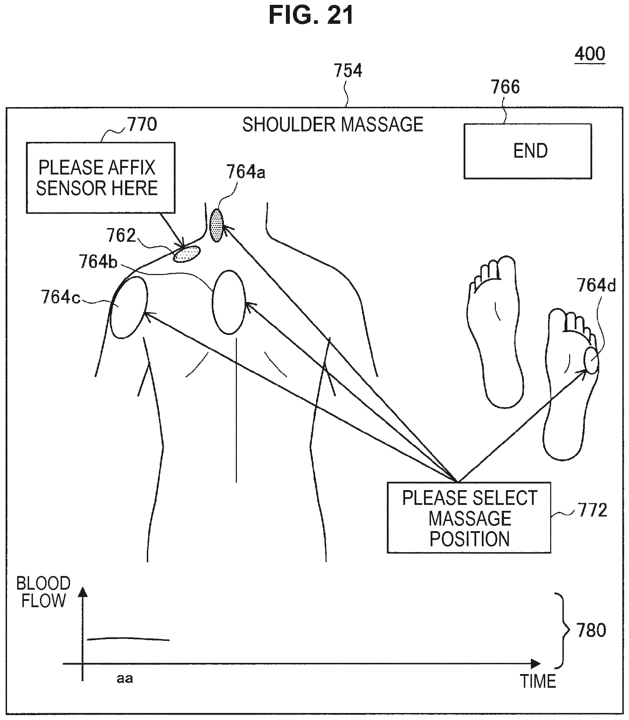

The measuring module 10 measures the blood flow velocity V of the measured person, and the presenting section 400 presents the blood flow velocity measured by the measuring module 10. Further, the measuring module 10 measures an elapsed time from the measurement start, and measures the blood flow velocity V of the measured person each time a predetermined period of time (for example, several seconds) elapses from the measurement start, and the presenting section 400 presents the blood flow velocity measured by the measuring module 10. For example, a screen 754 presented by the information presenting apparatus 40 at this time is illustrated in FIG. 21. The screen 754 includes a mark 764 indicating the treatment position selected by the user and another mark 764 indicating the treatment position not selected by the user. Specifically, the information presenting apparatus 40 displays the mark 764a indicating the treatment position selected by the user with a color, brightness, a blinking pattern, or the like different from those of the other marks 764b to 764d. Further, the screen 754 also includes a graph 780 indicating the change in the blood flow velocity of the measured person with respect to the elapsed time since the measuring module 10 starts the blood flow velocity measurement. Further, in the graph 780, a name of the treatment position selected by the user may be displayed together with the change in the blood flow velocity. For example, in the graph 780 of FIG. 21, "aa" is displayed as the name of the body part corresponding to the mark 764a indicating the treatment position selected by the user.

### (Step S311)

In a case in which the information presenting apparatus 40 receives an input manipulation for the user to change the treatment position as the user performs the manipulation on other marks 764 (the marks 764b to 764d) in addition to the mark 764 (here, the mark 764a) manipulated in step S307, the process proceeds to step S313. On the other hand, in a case in which the user does not manipulate other marks 764, and the information presenting apparatus 40 does not receive the input manipulation for the user to change the treatment position, the process proceeds to step S315.

### (Step S313)

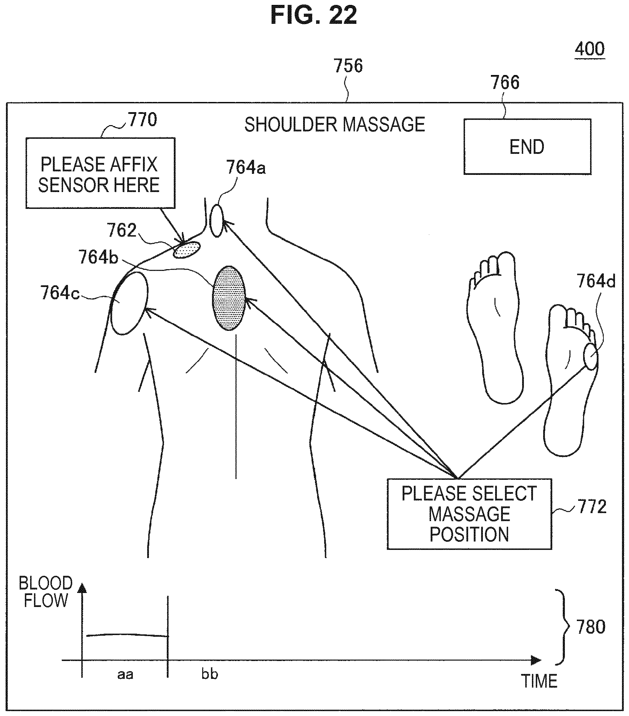

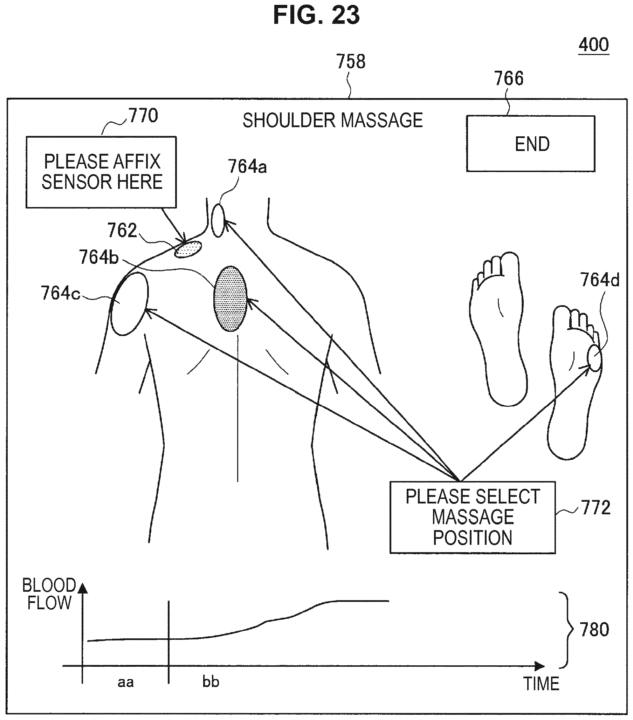

Similarly to step S309, the measuring module 10 measures the blood flow velocity V of the measured person, and the presenting section 400 presents the blood flow velocity measured by the measuring module 10 as, for example, a screen 756. At this time, similarly to step S309, as illustrated in FIG. 22, the information presenting apparatus 40 displays the mark 764b indicating the treatment position changed by the user in a form different from the other marks 764a, 764c, and 764d. Further, in the graph 780 included in the screen 756, the name of the treatment position selected by the user in step S311 may be displayed together with the blood flow velocity of the measured person measured by the measuring module 10. For example, in the graph 780 of FIG. 22, "bb" is displayed as the name of the body part corresponding to the mark 764b indicating the treatment position selected by the user. Further, in step S313, in a case in which the blood flow velocity V is consecutively measured, the information presenting apparatus 40 presents a screen 758 illustrated in FIG. 23.

### (Step S315)

In a case in which the information presenting apparatus 40 detects the manipulation signal corresponding to the manipulation indicating that the information processing is ended as the user performs a manipulation on the end button 766, the information processing ends. Further, in a case in which the information presenting apparatus 40 does not detect the manipulation signal, steps S309 to S315 described above may be repeated.

Further, the measuring module 10 in the present embodiment is not limited to a pad-like bandage type wearable apparatus but may be a wearable apparatus of another form such as a band-like bracelet type in accordance with the treatment position or the like.

As described above, in the present embodiment, since the blood flow velocity is measured during the treatment, and the measured blood flow velocity is presented, the user can confirm a part which has to be treated to increase the effect of the blood flow improvement during the treatment, and it is possible to review the treatment technique or the like suitable for the measured person. In other words, according to the present embodiment, it is possible to review behaviors that have an effect appropriate to the body such as the treatment method suitable for the subject by using the blood flow velocity measurement function by the wearable apparatus being worn. Further, when an exercise to improve the blood flow such as gymnastics, stretching, or the like is performed, it is preferable to use the measuring module 10 or the like as follows. For example, the measuring module 10 measures the blood flow velocity in a state in which the measured person takes a relaxing posture (for example, a recumbent position) before the above exercise, and the measuring module 10 measures the blood flow velocity in a state in which the measured person takes the relaxing posture again after the above exercise. By comparing and confirming the change in the blood flow velocity before and after the exercise measured in this manner using the information presenting apparatus 40 or the like, the user can confirm the effect of exercise. Further, the measurement result of the blood flow velocity during the treatment may be associated with the measured person to form a database, and the database may be used in, for example, a massage apparatus that automatically performs massage.

### <<6. Information processing method according to fourth embodiment of present disclosure>>

Next, an information processing method according to a fourth embodiment of the present disclosure will be described. In the first to third embodiments, in order to measure the blood flow velocity of the measured person accurately, it is preferable to fix the measuring module 10 to the body of the measured person to the extent that the blood flow of the measured person is not disturbed. However, in a case in which the measuring module 10 is loosely fixed or in a case in which dust adheres to the adhesive layer 116 installed in the measuring module 10, and the adhesion to the skin is weakened, the installation position of the measuring module 10 may deviate from the measurement region during the measurement. In this case, the blood flow velocity of the measured person is unable to be measured accurately. In this regard, in the present embodiment, an installation state of the measuring module 10 is determined using a pulse wave obtained by measuring the blood flow velocity of the measured person. Then, in the present embodiment, in a case in which it is determined that the fixing of the measuring module 10 is loose, the user is encouraged to fix the measuring module 10 so that the measuring module 10 does not move.

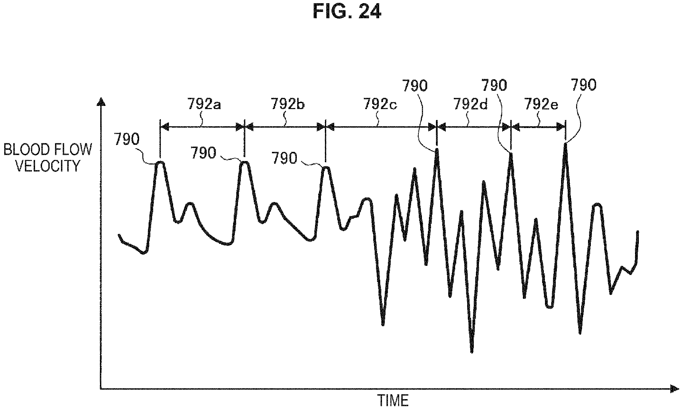

The present embodiment will be specifically described below with reference to FIG. 24. FIG. 24 is an explanatory diagram for describing a fourth embodiment of the present disclosure, and specifically illustrates a temporal change in the blood flow velocity detected by measuring module 10. The blood flow velocity varies regularly depending on the pulse of the measured person, and a peak 790 caused by the pulse of the measured person repeatedly appears in the temporal change in the blood flow velocity as illustrated in FIG. 24. In the following description, waveforms interposed between the two peaks 790 illustrated in FIG. 24 are referred to as pulse waves 792 (792a to 792e) which are waveforms obtained by a single pulse. In the present embodiment, the calculating apparatus 30 can determine the fixed state of the measuring module 10 by comparing the shape of each pulse wave 792.

Normally, pulse waves having a similar shape consecutively appear in the temporal change in the blood flow velocity in a state in which there is no factor (for example, sudden movement starts or the like) in which the blood flow velocity of the measured person changes dramatically. However, in a case in which the measuring module 10 is not fixed and deviates from the measurement region, it causes a pulse wave of a disturbed shape to appear in the above signal. For example, in the example illustrated in FIG. 24, there are waveforms substantially similar to the pulse wave 792a and the pulse wave 792b, and the pulse wave 792a and the pulse wave 792b are examples of the pulse wave obtained in a case in which the measured person is in the rest state. On the other hand, the pulse wave 792a, the pulse wave 792c, the pulse wave 792d, and the pulse wave 792e have shapes different from one another, and the pulse waves 792c to 792e are examples of the pulse wave in a case in which it is disturbed due to some factors. In this regard, in a case in which the calculating apparatus 30 is unable to consecutively obtain the pulse wave such as the pulse wave 792a, it is determined that the same pulse wave is unable to be consecutively obtained because the measuring module 10 deviates from the measurement region, and the fixing of the measuring module 10 is loose. Further, on the basis of the above determination of the calculating apparatus 30, the information presenting apparatus 40 performs a presentation for encouraging the user to fix the measuring module 10 so that the measuring module 10 does not move. Further, when the measuring room 10 is mounted, the measured person may be asked to move the installation position (for example, the arm, the leg, or the like), and the calculating apparatus 30 may checks whether or not the pulse wave is disturbed by the motion of the measured person and determine the fixing state of the measuring module 10.

As described above, in the present embodiment, the installation state of the measuring module 10 is determined using the pulse wave obtained by measuring the blood flow velocity of the measured person. Then, in a case in which the fixing of the measuring module 10 is determined to be loose, the user is encouraged to fix the measuring module 10 so that the measuring module 10 does not move. Therefore, according to the present embodiment, in order to accurately measure the blood flow velocity of the measured person, it is possible to ensure that the measuring module 10 is properly fixed to the body of the measured person. Further, the present embodiment is not limited to determining the installation state of the measuring module 10 using the pulse wave obtained by measuring the blood flow velocity of the measured person, and the installation state of the measuring module 10 may be determined using interference light or the like obtained when the blood flow velocity of the measured person is measured.

### <<7. Information processing method according to fifth embodiment of present disclosure>>

Next, an information processing method in accordance with a fifth embodiment of the present disclosure will be described. In the first to third embodiments, the measuring module 10 is a wearable apparatus worn on the measured person and is thus preferably compact. Further, in order to reduce the power capacity of the measuring module 10, it is preferable that the power consumption of the measuring module 10 be suppressed as much as possible. In this regard, in the following description, the measuring module 10 according to the present embodiment which can suppress power consumption will be described.

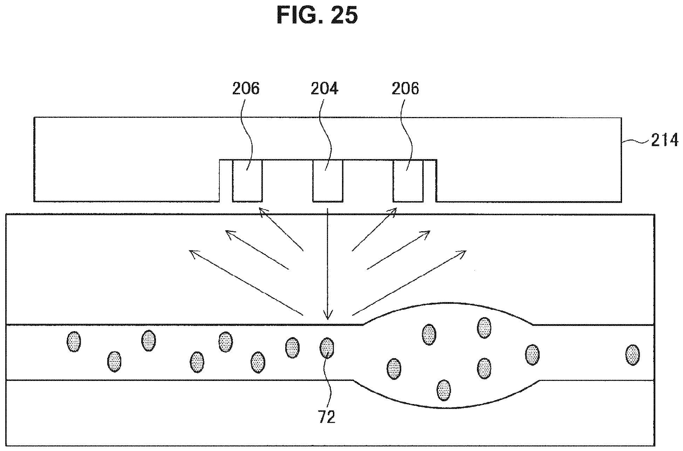

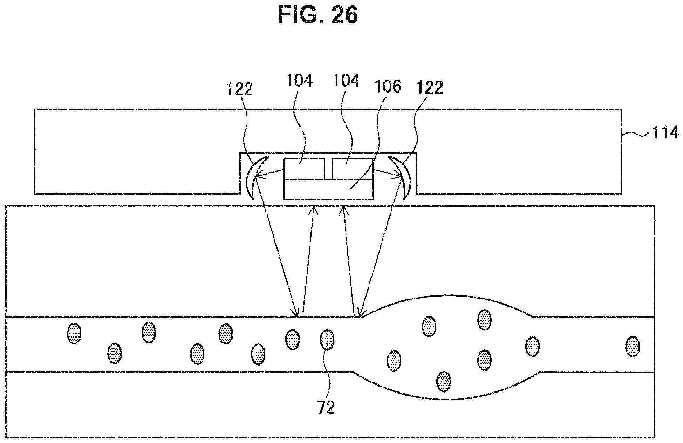

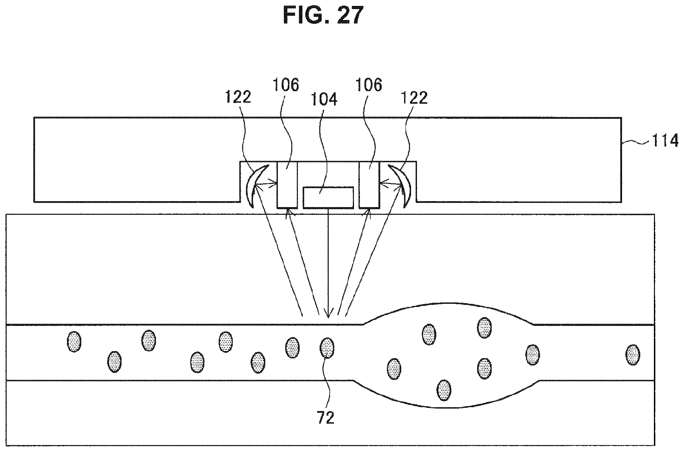

The present embodiment will be specifically described below with reference to FIGS. 25 to 27. FIG. 25 is an explanatory diagram for describing a configuration of a measuring unit 214 of a measuring module 20 of a comparative example. FIG. 26 is an explanatory diagram for describing a configuration of a measuring unit 114 of a measuring module 10 according to the present embodiment. FIG. 27 is an explanatory diagram for describing a configuration of a measuring unit 114 of a measuring module 10 in accordance with a modified example of the present embodiment.

As illustrated in FIG. 25, the measuring unit 214 of the comparative example includes an irradiating section 204 and two detecting sections 206, and the irradiating section 204 and the two detecting sections 206 are arranged in a row along the skin on the surface of the measurement region of the measured person. In the comparative example, irradiation light with which the measurement region of the measured person is irradiated from the irradiating section 204 is scattered by a scattering material 72 in the measurement region and spreads in all directions, as illustrated in FIG. 25. Since the measuring unit 214 is installed on the body of the measured person, the size of the measuring unit 214 is restricted, and the size of the detecting section 206 and the number of installations of the detecting section 206 are restricted as well. Therefore, a few small detecting sections 206 can detect only a part of the scattered light spreading in all directions as illustrated in FIG. 25.

On the other hand, in the measuring unit 114 according to the present embodiment, as illustrated in FIG. 26, in addition to the irradiating section 104 and the detecting section 106, a reflecting plate 122 that controls directivity of the irradiation light irradiated from the irradiating section 104 and guides the irradiation light to be directed toward the measurement region of the measured person is installed. Specifically, as illustrated in FIG. 26, the reflecting plate 122 can reflect the irradiation light that is irradiated from the irradiating section 104 and reaches the reflecting plate 122 so that the irradiation light reaches the measurement region. In other words, the reflecting plate 122 can be a pseudo light source that directly irradiates light toward the measurement region of the measured person. Therefore, since the irradiating section 104 need not be installed to face the measurement region in order to directly irradiate the measurement region with light, instead, it is possible to increase the surface opposing the measurement region of the detecting section 106 installed to face the measurement region. Further, since it is possible to increase the surface facing the detecting section 106, the detecting section 106 can detect more scattered light which are scattered in the measurement region and spreading in all directions. Therefore, since the detecting section 106 can detect more scattered light even in a case in which the intensity of the irradiation light is lowered in order to suppress the power consumption of the irradiating section 104, it is possible to measure the blood flow velocity efficiently and accurately. Further, since the irradiating section 104 need not be installed to face the measurement region, the irradiating section 104 may be stacked above the detecting section 106 as illustrated in FIG. 26. When the irradiating section 104 is stacked on the detecting section 106 as described above, it is possible to reduce an area of a surface facing a measurement region of a space storing the irradiating section 104 and the detecting section 106, and the measuring module 10 can be made compact.

Further, in the present embodiment, the irradiating section 104 is not limited to being stacked above the detecting section 106. For example, the detecting section 106 may be stacked above the irradiating section 104 to replace the irradiating section 104 and the detecting section 106 in FIG. 26. In this case, the irradiating section 104 facing the measurement region irradiates the measurement region with light. The irradiated light is scattered by the scattering material 72 of the measurement region as described above and spreads in all directions, but the scattered light spreading in all directions is guided to the detecting section 106 by the reflecting plate 122. Thus, the detecting section 106 can detect the scattered light which is scattered in the measurement region and spreading in all directions. In other words, in this case, since the detecting section 106 is stacked over the irradiating section 104, the detecting section 106 can measure the blood flow velocity efficiently and accurately while making the measuring module compact.

Further, the reflecting plate 122 according to the present embodiment may be formed of a metal material capable of reflecting the irradiation light, a surface-processed resin material, or the like, and a shape thereof is not limited as long as it is a shape capable of guiding the irradiation light to the measurement region. For example, the reflecting plate 122 may have a circular arc-like curved surface as a reflecting surface or may have a shape similar to a hollow hemisphere surrounding the irradiating section 104 and the detecting section 106 which are stacked, and in the latter case, an inner surface of the hemisphere functions as the reflection surface.

Further, the above embodiments may be modified as follows. In the measuring unit 114 of the modified example, as illustrated in FIG. 27, in addition to the irradiating section 104 and the detecting section 106, a reflecting plate 122 that controls directivity of the scattered light which is scattered by the scattering material 72 of the measurement region and spreading in all directions and guides the scattered light to be directed toward detecting section 106 is installed. Specifically, as illustrated in FIG. 27, the reflecting plate 122 can reflect the scattered light which is scattered in the measurement region and reaches the reflecting plate 122 so that the scattered light reaches the detecting section 106. As a result, the detecting section 106 can not only detect the scattered light scattered in the measurement region directly but also detect the scattered light guided by the reflecting plate 122. Therefore, since the detecting section 106 can also detect more scattered lights even in a case in which the intensity of irradiation light is lowed in order to suppress the power consumption of the irradiating section 104, it is possible to measure the blood flow velocity efficiently and accurately. Further, the reflecting plate 122 is not limited to the reflecting plate which guides light through the above-described path and may be a reflecting plate which reflects transmitted light passing through the measurement region and guides the transmitted light to be directed toward the detecting section 106.

As described above, according to the present embodiment, the installation positions of the irradiating section 104 and the detecting section 106 are further reviewed using the reflecting plate 122, and thus it is possible to obtain the measuring module 10 which can measure the blood flow velocity effectively and accurately while suppressing the power consumption.

### <<8. Measurement method according to sixth embodiment of present disclosure>>

Next, an information processing method according to a sixth embodiment of the present disclosure will be described. In the first to third embodiments, the measuring module 10 may measure the blood flow velocity of the measured person over a long period of time. Therefore, it is preferable that the power consumption of the measuring module 10 according to the present embodiment be further suppressed. Further, most of the power consumption in the measuring module 10 is performed by the irradiating section 104. In this regard, in the following description, a measurement method according to the present embodiment capable of suppressing the power consumption in the irradiating section 104 will be described.

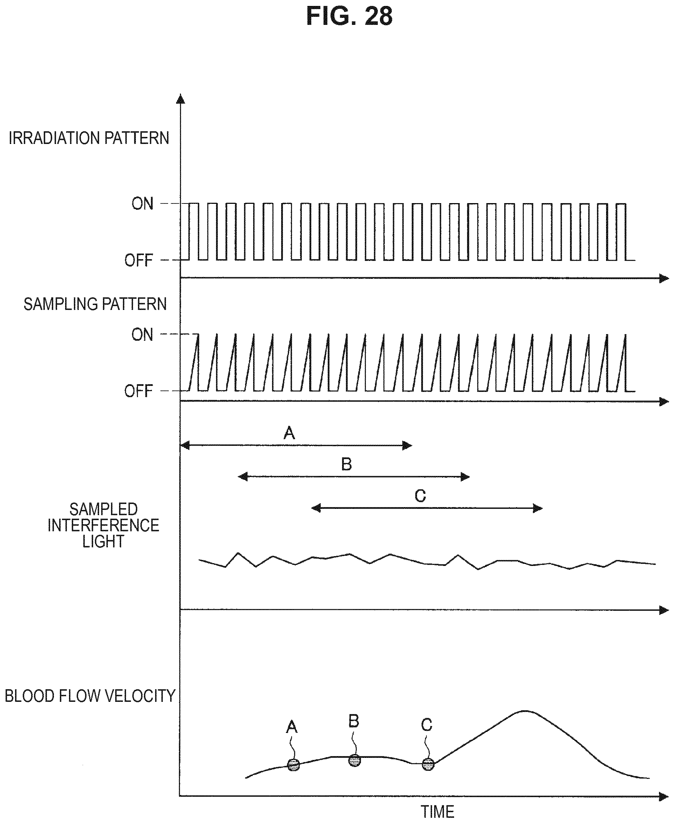

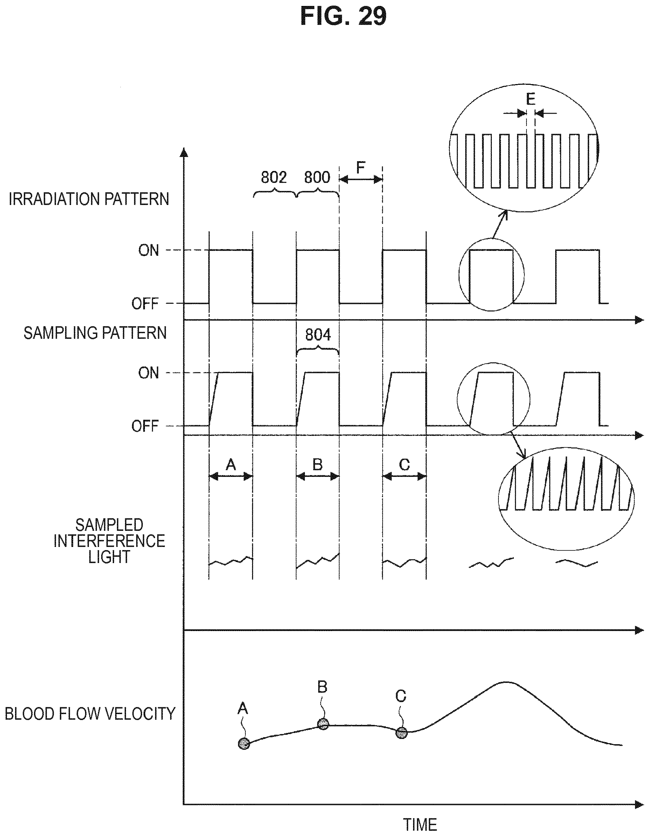

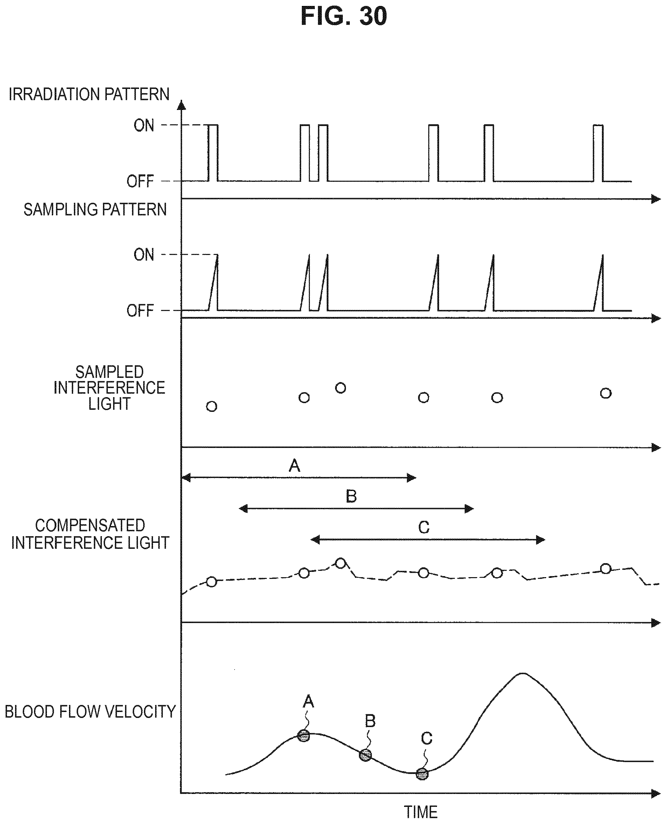

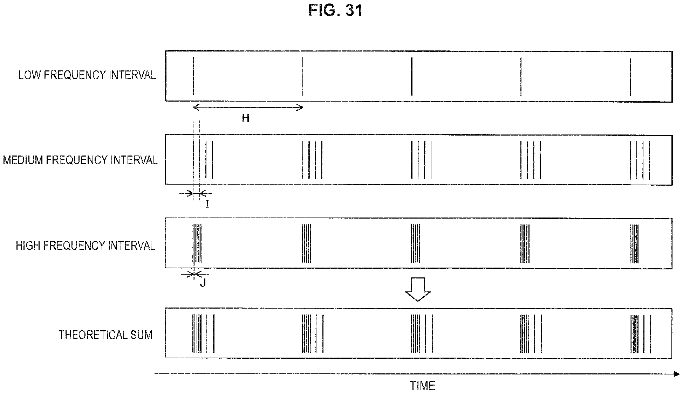

A measurement method according to the present embodiment will be specifically described below with reference to FIGS. 28 to 31. FIG. 28 is an explanatory diagram for describing a measurement method of a comparative example. FIG. 28 schematically illustrates an irradiation pattern of the irradiating section 104, a reading (sampling) pattern of the detecting section 106, a temporal change in interference light obtained by the detecting section 106, and a temporal change in the blood flow velocity calculated from the interference light according to the comparative example in order from an upper part from a lower part. FIG. 29 is an explanatory diagram for describing a measurement method according to the present embodiment. Specifically, FIG. 29 schematically illustrates an irradiation pattern of the irradiating section 104, a reading (sampling) pattern of the detecting section 106, a temporal change in interference light obtained by the detecting section 106, and a temporal change in the blood flow velocity calculated from the interference light according to the present embodiment in order from an upper part from a lower part. Further, in FIG. 29, an enlarged view of a part of the irradiation pattern illustrated in a first row and an enlarged view of a part of the reading pattern illustrated in a second row are also illustrated. FIG. 30 is an explanatory diagram for describing a first modified example of the present embodiment. Specifically, FIG. 30 schematically illustrates an irradiation pattern of the irradiating section 104, a reading (sampling) pattern of the detecting section 106, an interference light obtained by the detecting section 106 according to a first modified example from a first row to a third row. Further, a temporal change in the interference light obtained by performing a process of compensating data of the interference light in the third row of FIG. 30 is schematically illustrated in a fourth row of FIG. 30, and a temporal change in the blood flow velocity calculated from the compensated interference light is schematically illustrated in a fifth row of FIG. 30. Further, FIG. 31 is an explanatory diagram for describing a second modified example of the present embodiment, and in detail, schematically illustrates a sampling timing in detecting section 106. Further, in the following description, "sampling" refers to reading charges charged in the detecting section 106 since the interference light is detected.

First, the measurement method of the comparative example will be described with reference to FIG. 28. As illustrated in FIG. 28, the irradiation pattern of the irradiating section 104 has a rectangular wave, and in detail, the irradiating section 104 irradiates light in periods of upper flat portions (ON) (an irradiation period). On the other hand, the irradiating section 104 pauses the irradiation in periods of lower flat portions (OFF) (a pause period). Further, as indicated by the reading pattern of the detecting section 106, the reading timing of the detecting section 106 is synchronized with the irradiation period of the irradiating section 104. Specifically, as described above, charges are charged in the detecting section 106 including a photodiode or the like in the irradiation period, and the charges charged in the detecting section 106 are read (sampled) at the timing at which the irradiating section 104 pauses the irradiation. In other words, the charges charged in the detecting section 106 are read out in peak portions of the reading pattern in the second row of FIG. 28. Further, a temporal change in the interference light illustrated in the third row of FIG. 28 is obtained by consecutively plotting the read sampling results over the measurement time. Then, it is possible to obtain one value of the blood flow velocity for each section by calculating an auto-correlation function in certain sections (for example, an A section, a B section, and a C section illustrated in the third row of FIG. 28) in the temporal change in the interference light. For example, it is possible to obtain a blood flow velocity value A (a plot A illustrated in the fourth row of FIG. 28) from the auto-correlation function of the A section illustrated in the third row of FIG. 28. A temporal change in the blood flow velocity illustrated in the fourth row of FIG. 28 is obtained by consecutively plotting the blood flow velocity obtained as described above over the measurement time.

As described above, in the comparative example, the temporal change in the blood flow velocity illustrated in the fourth row of FIG. 28 is acquired by repeatedly performing the irradiating by the irradiating section 104. Therefore, it is difficult to suppress the power consumption in the irradiating section 104 because the irradiating section 104 repeats the irradiation consecutively.

In this regard, in the present embodiment, as illustrated in the first row of FIG. 29, the control section 108 controls the irradiating section 104 such that the irradiation pattern of the irradiating section 104 becomes a pattern in which a section 800 in which the irradiating section 104 regularly repeats the irradiation and a section 802 in which the irradiation is paused appear alternately. In this case, it is possible to suppress the power consumption in the irradiating section 104.

Specifically, as illustrated in the first row of FIG. 29, the section 800 in which the irradiating section 104 regularly repeats the irradiation and the section 802 in which the irradiation is paused appear alternately in the irradiation pattern of the irradiating section 104. In the section 800 in which the irradiating section 104 regularly repeats the irradiation, the irradiating section 104 intermittently irradiates the irradiation light at a first interval E as illustrated in the enlarged view of the section 800. Further, the section 800 is repeated at a second interval F corresponding to the section 802 in which the irradiation is paused. The second interval F is set to be longer than the first interval E. Accordingly, since the irradiation period in which the irradiation is performed by the irradiating section 104 can be reduced as compared with the irradiation pattern of the comparative example illustrated in the first row of FIG. 28, the power consumption in the irradiating section 104 can be suppressed.

Further, in the comparative example, the temporal change in the consecutive interference light illustrated in the third row of FIG. 28 can be obtained, whereas in the present embodiment, the temporal change in the intermittent interference light illustrated in the third row of FIG. 29 is obtained using the irradiation pattern illustrated in the first row of FIG. 29. However, although it is the temporal change in the intermittent interference light, it is possible to one value of the blood flow velocity for each section by calculating the auto-correlation function using the interference light of the sections in which there is interference light (for example, the A section, the B section, and the C section illustrated in the third row of FIG. 29). For example, the blood flow velocity value A (plot A illustrated in the fourth row in FIG. 29) can be obtained from the auto-correlation function of the A section illustrated in the third row of FIG. 29. A temporal change in the blood flow velocity illustrated in the fourth row of FIG. 29 is obtained by consecutively plotting the blood flow velocity obtained as described above over the measurement time.

Further, as can be seen from FIGS. 28 and 29, in the present embodiment, since the blood flow velocity is calculated for each section in which there is interference light from the temporal change in the intermittent interference light, a plurality of sections used for the calculation of the blood flow velocity do not overlap, and the length of each section is reduced. On the other hand, in the comparative example, a plurality of sections used for the calculation of the blood flow velocity overlap, and the length of each section is also long. As described above, the section of the interference light used for the calculation of the blood flow velocity differ between the present embodiment and the comparative example. However, in a case in which the first to third embodiments are applied, there is no significant difference in the measurement accuracy or the like between the present embodiment and the comparative example, and in the present embodiment, it is possible to maintain the measurement accuracy equivalent to that of the comparative example.

As described above, in the present embodiment, the irradiation pattern of the irradiating section 104 becomes a pattern in which the section 800 in which the irradiating section 104 regularly repeats the irradiation and the section 802 in which the irradiation is paused appear alternately. Accordingly, according to the present embodiment, it is possible to suppress the power consumption in the irradiating section 104 while maintaining the excellent measurement accuracy.

### <First modified example>

Further, the above embodiments may be modified as follows. In a first modified example to be described below, the interference light is sampled at a random timing, and the sampled interference light is processed, and thus the sampled interference light data is compensated, and the temporal change in the consecutive interference light is acquired. Then, in the present first modified example, the calculating apparatus 30 can obtain the value of the blood flow velocity for each section by calculating the auto-correlation function in each section in the temporal change in the compensated interference light as in the comparative example.

Since the interference light obtained by scattering from the blood flow includes a waveform to be repeated as a time elapse, the data can be compensated by machine learning using data of the interference light of unequal intervals which are randomly sampled.

Specifically, in the present first modified example, the detecting section 106 performs sampling of the interference light at an interval having randomness as illustrated in a second row of FIG. 30. Further, the irradiating section 104 is controlled by the control section 108 such that that the irradiation of the irradiation light is repeated at an interval having randomness as illustrated in a first row of FIG. 30 to be synchronized with the sampling timing of the detecting section 106. As described above, in the present first modified example, the irradiation interval of the irradiation pattern of the irradiating section 104 is changed to a random length of time, and thus the irradiation period of the irradiating section 104 can be reduced, and the power consumption in the irradiating section 104 can be reduced.

Then, a third row of FIG. 30 is obtained by plotting the interference light read by the detecting section 106 at the interval having the randomness over time. Further, the compensation is performed using the data of the interference light of the unequal interval illustrated in the third row of FIG. 30, and the temporal change in the consecutive interference light illustrated in a fourth row of FIG. 30 is obtained. Then, as in the comparative example, it is possible to obtain the value of the blood flow velocity (for example, a plot A, a plot B, and a plot C illustrated in a fifth row of FIG. 30) for each section and obtain the temporal change in the blood flow velocity by calculating the auto-correlation function in each section (for example, the A section, the B section, and the C section illustrated in the fourth row of FIG. 30) in the temporal change in the interference light obtained by the compensation.

As described above, in the present first modified example, it is possible to obtain the temporal change in the blood flow velocity from the interference light data sampled at the interval with the randomness using the compensation by the machine learning. Therefore, in accordance with the present first modified example, it is possible to reduce the irradiation period of the irradiating section 104 and suppress the power consumption in the irradiating section 104.

### <Second modified example>

As can be understood from the above embodiments and the first modified example, if the interference light is sampled with a sampling interval corresponding to the range of the blood flow velocity desired to be analyzed, it is possible to accurately obtain the temporal change in the blood flow velocity even when the interference light is not consecutively sampled. In this regard, in the following description, a measurement method capable of reducing the irradiation period of the irradiating section 104 and suppress the power consumption in the irradiating section 104 while performing sampling to correspond to a range of a plurality of blood flow velocities will be described as a second modified example.

For example, the range (content) of the blood flow velocity desired to be analyzed and a sampling interval corresponding thereto are as follows. In a case in which it is desired to detect a pulse from the blood flow velocity, a sampling interval of about several hundred of Hz is set in accordance with the pulse (in the following description, it is referred to as a low frequency interval). Further, in a case in which it is desired to detect a temporal change in a slow blood flow velocity in a blood vessel located near the skin surface of the measurement region of the measured person, a sampling interval of around several kHz is set (in the following description, it is referred to as a medium frequency interval). Further, in a case in which it is desired to detect a temporal change in a fast blood flow velocity in the blood vessel at a deep position from the skin surface of the measurement region of the measured person, a sampling interval of around several tens of kHz is set (in the following description, it is referred to as a high frequency interval).

A sampling timing in the low frequency interval, a sampling timing in the medium frequency interval, a sampling timing in the high frequency interval, and a sampling timing based on a theoretical sum thereof are illustrated from an upper part from a lower part of FIG. 31. A horizontal axis indicates a time, and the interference light is sampled at each of timings indicated by vertical lines. Further, in FIG. 31, in order to make it easy to see, sampling timings which are coarser (longer in interval) than actual sampling timings are schematically illustrated. Specifically, the low frequency interval for detecting the pulse wave illustrated in the first row of FIG. 31 has a sampling interval H. Further, the medium frequency interval for detecting the blood flow velocity in the blood vessel located near the skin surface of the measurement region of the measured person illustrated in the second row of FIG. 31 has a sampling interval I. The sampling interval I is shorter than the sampling interval H depending on a detection target. Further, the high frequency interval for detecting the blood flow velocity in the blood vessels at a deep position from the skin surface of the measurement region of the measured person illustrated in the third row of FIG. 31 has a sampling interval J. The sampling interval J is shorter than the sampling interval H and the sampling interval I depending on a detection target.

Then, if times of initial sampling timings are set to be the same, and a logical sum of these sampling timings is obtained, a sampling pattern illustrated in the fourth row in FIG. 31 is obtained. The interference light sampled at the sampling timing in the fourth row of FIG. 31 is set to be the same as the interference light sampled at each of the sampling timings illustrated from the first to the third rows of FIG. 31. Therefore, in the present modified example, the interference light is sampled with a combined sampling timing illustrated in a fourth row of FIG. 31, and thus it is possible to perform sampling for a range of a plurality of blood flow velocities corresponding to the low frequency interval, the medium frequency interval, and the high frequency interval. Further, since the sampling is not consecutive, and the sampling pattern is intermittent as can be understood from the fourth row of FIG. 31, an intermittent pattern is obtained even in the irradiation period of the irradiating section 104 synchronized with the irradiation pattern corresponding to the sampling pattern. Therefore, according to the present second modified example, it is possible to reduce the irradiation period of the irradiating section 104 and suppress the power consumption in the irradiating section 104.

As described above, in the present second modified example, since the sampling intervals corresponding to the range of a plurality of blood flow velocities are combined, it is possible to reduce the irradiation period of the irradiating section 104 and suppress the power consumption in the irradiating section 104 while performing sampling to correspond to the range of a plurality of blood flow velocities.

### <<9. Hardware configuration>>

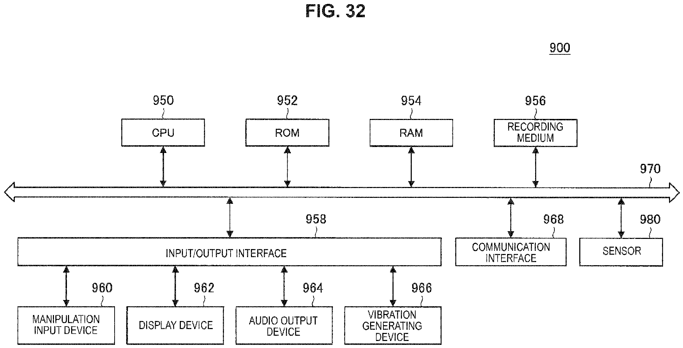

FIG. 32 is an explanatory diagram illustrating an example of a hardware configuration of an information processing apparatus 900 according to the present embodiment. In FIG. 32, the information processing apparatus 900 is an example of a hardware configuration in a case in which the measuring module 10, the calculating apparatus 30, the information presenting apparatus 40, and the posture detecting apparatus 50 are formed as an integrated wearable apparatus.

The information processing apparatus 900 includes, for example, a CPU 950, a ROM 952, a RAM 954, a recording medium 956, an input/output interface 958, and a manipulation input device 960. Further, the information processing apparatus 900 includes a display device 962, an audio output device 964, a vibration generating device 966, a communication interface 968, and a sensor 980. Further, in the information processing apparatus 900, for example, the respective components are connected by a bus 970 serving as a data transmission path.

### (CPU 950)

For example, the CPU 950 functions as a control section (for example, the control sections 108 and 402) which is constituted by one or more processors constituted by an arithmetic circuit such as, for example, a CPU, various kinds of processing circuits, or the like and controls the information processing apparatus 900 in general. Further, the CPU 950 undertakes, for example, the functions of the calculating section 300, the comparing section 304, the analyzing section 502, and the like in the information processing apparatus 900.

### (ROM 952 and RAM 954)

The ROM 952 stores a program, control data such as operation parameters, and the like used by the CPU 950. The RAM 954 temporarily stores, for example, a program executed by the CPU 950.

### (Recording medium 956)

The recording medium 956 functions as the storage section 302, and stores, for example, data related to the information processing method according to the present embodiment and various data such as various kinds of applications. Here, examples of the recording medium 956 include a magnetic recording medium such as a hard disk and a non-volatile memory such as a flash memory. Further, the recording medium 956 may be removable from the information processing apparatus 900.

### (Input/output interface 958, manipulation input device 960, display device 962, audio output device 964, and vibration generating device 966)

The input/output interface 958 connects, for example, the manipulation input device 960, the display device 962, and the like. Examples of the input/output interface 958 include a universal serial bus (USB) terminal, a digital visual interface (DVI) terminal, a high-definition multimedia interface (HDMI) (registered trademark) terminal, and various kinds of processing circuits.

The manipulation input device 960 functions as a manipulating unit (not illustrated), is installed in, for example, the information processing apparatus 900, and is connected with the input/output interface 958 in the information processing apparatus 900. Examples of the manipulation input device 960 include a button, a directional key, a rotary selector such as a jog dial, a touch panel, and a combination thereof.

The display device 962 functions as the presenting section 400 including the display apparatus, and is installed, for example, in the information processing apparatus 900 and connected with the input/output interface 958 in the information processing apparatus 900. Examples of the display device 162 include a liquid crystal display, an organic electro-luminescence (EL) display, and an OLED display.

The audio output device 964 functions as the presenting section 400 including the audio output apparatus, and is installed for example, in the information processing apparatus 900 and connected with the input/output interface 958 in the information processing apparatus 900. As the audio output device 964, a speaker or the like may be used.

The vibration generating device 966 functions as the presenting section 400 including the vibration generating apparatus, and is installed, for example, in the information processing apparatus 900 and connected with the input/output interface 958 in the information processing apparatus 900. As the vibration generating device 966, a vibrator including a small motor or the like is used.

Further, it goes without saying that the input/output interface 958 can also be connected with an external device such as a manipulation input device (for example, a keyboard, a mouse, or the like) outside the information processing apparatus 900 or an external display device.

### (Communication interface 968)

A communication interface 968 is a communication unit installed in the information processing apparatus 900 and functions as a communication section (not illustrated) for performing wireless or wired communication with an external apparatus such as a server via a network (or directly). Here, examples of the communication interface 968 include a communication antenna and a radio frequency (RF) circuit (wireless communication), an IEEE 802.15.1 port and a transceiving circuit (wireless communication), an IEEE 802.11 port and a transceiving circuit (wireless communication), and a local area network (LAN) terminal and a transceiving circuit (wired communication).

### (Sensor 980)

The sensor 980 is a sensor that functions as the measuring module 10 and detects the blood flow velocity in accordance with an arbitrary scheme capable of detecting the blood flow velocity. The sensor 980 includes, for example, the irradiating section 104 that emits light and the detecting section 106 that generates a signal in response to received light. The irradiating section 104 includes, for example, one or more light sources such as a laser. Further, the detecting section 106 also includes, for example, a photodiode, an amplifier circuit, a filter circuit, and an analog-to-digital converter.

Further, the sensor 980 may have a function of the posture detecting apparatus 50. In this case, the sensor 980 may include one or more sensors capable of detecting a motion such as an acceleration sensor, a gyro sensor, or the like. Further, the sensor included in the sensor 980 is not limited to the above example. For example, the sensor 980 may be any sensor capable of obtaining an arbitrary sensing result which can be used to detect the posture of the user such as a geomagnetic sensor, an imaging device, or the like.

Further, the hardware configuration of the information processing apparatus 900 is not limited to the configuration illustrated in FIG. 32.

For example, in this case in which the information processing apparatus 900 communicates with an external apparatus or the like via an external communication device connected thereto or in a case in which the information processing apparatus 900 is configured to perform standalone processing, the information processing apparatus 900 may not include the communication interface 968. Further, the communication interface 968 may have a configuration capable of communicating with one or more external apparatuses in accordance with a plurality of communication schemes.

Further, the information processing apparatus 900 may have a configuration which does not include, for example, the recording medium 956, the manipulation input device 960, the display device 962, or the like.

While the information processing apparatus has been described as the present invention, the present embodiment is not limited to such an embodiment. The present embodiment can be applied to various devices capable of performing processing related to the information processing method according to the present embodiment such as a communication apparatus such as a cellular phone, a vehicle such as an automobile, or the like.

Further, the information processing apparatus according to the present embodiment may be applied to a system including a plurality of apparatuses based on a connection to a network (or communication between respective apparatuses) as in cloud computing or the like. In other words, the information processing apparatus according to the present embodiment can also be realized as, for example, an information processing system which performs processing related to the information processing method according to the present embodiment through a plurality of apparatuses.

The example of the hardware configuration of the information processing apparatus 900 has been described above. Each of the components may be constituted using a general-purpose member or may be constituted by hardware specialized for the function of each component. Such a configuration can be appropriately changed in accordance with a technical level at the time of implementation.

### <<10. Supplement>>

Further, the embodiments of the present disclosures described above may include, for example, a program causing a computer to function as the information processing apparatus according to the present embodiment and a non-transitory tangible medium having a program recorded therein. Further, the program may be distributed via a communication line such as the Internet (including wireless communication).

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An information processing apparatus, including:
   a comparing section configured to perform a comparison using a blood flow velocity of a measured person in a predetermined state and a blood flow velocity of the measured person in a state other than the predetermined state; and
   an information presenting section configured to present information corresponding to a comparison result of the comparing section.
(2) The information processing apparatus according to (1), in which the information presenting section includes at least one of an audio output apparatus, a vibration generating apparatus, or a display apparatus.
(3) The information processing apparatus according to (1) or (2), in which the information presenting section presents information for guiding a user of the information processing apparatus to perform a predetermined behavior as the information corresponding to the comparison result.
(4) The information processing apparatus according to (3), in which the user includes the measured person.
(5) The information processing apparatus according to any one of (1) to (4), further including:
   a posture detecting section configured to detect a posture of the measured person.
(6) The information processing apparatus according to any one of (1) to (4), further including:
   a state detecting section configured to detect a state of the measured person.
(7) The information processing apparatus according to any one of (1) to (6), further including:
   a measuring section which is installed on a measurement region of the measured person and configured to measure the blood flow velocity of the measured person.
(8) The information processing apparatus according to (7), in which the comparing section determines an installation state of the measuring section using a pulse wave obtained from the blood flow velocity of the measured person.
(9) The information processing apparatus according to (7) or (8), in which the measuring section further includes
   an irradiating section configured to irradiate the measurement region with irradiation light,
   a detecting section configured to detect light from the measurement region, and
   a reflecting plate configured to guide the irradiation light to the measurement region or guide the light from the measurement region to the detecting section.
(10) The information processing apparatus according to (9), in which the irradiating section is stacked above the detecting section.
(11) The information processing apparatus according to (9) or (10), in which the reflecting plate includes a curved surface.
(12) The information processing apparatus according to any one of (9) to (11), further including:
   a control section configured to control the irradiating section such that an irradiation pattern in which the irradiating section intermittently irradiates the irradiation light at a first interval is repeated at a second interval longer than the first interval.
(13) The information processing apparatus according to any one of (9) to (11), further including:
   a control section configured to control the irradiating section such that an irradiation interval of the irradiating section is changed to a random length of time.
(14) The information processing apparatus according to any one of (9) to (11), further including:
   a control section configured to control the irradiating section in accordance with an irradiation pattern corresponding to a sampling pattern obtained by combining a plurality of sampling intervals corresponding to a plurality of different detection targets.
(15) An information processing method, including:
   performing a comparison using a blood flow velocity of a measured person in a predetermined state and a blood flow velocity of the measured person in a state other than the predetermined state; and
   presenting information corresponding to a comparison result obtained through the comparison.
(16) A program causing a computer to implement:
   a function of performing a comparison using a blood flow velocity of a measured person in a predetermined state and a blood flow velocity of the measured person in a state other than the predetermined state; and
   a function of presenting information corresponding to a result of the comparison.

### Reference Signs List

- 1: information processing system
- 10, 20: measuring module
- 30: calculating apparatus
- 40: information presenting apparatus
- 40a: vibration generating apparatus
- 50: posture detecting apparatus
- 60: pressurizer
- 70: stationary tissue
- 72: scattering material
- 74: red blood cell
- 104, 204: irradiating section
- 106, 206: detecting section
- 108, 402: control section
- 110: band portion
- 112: control unit
- 114,214: measuring unit
- 116: adhesive layer
- 118: magnet
- 120: pad portion
- 122: reflecting plate
- 300: calculating section
- 302: storage section
- 304: comparing section
- 400: presenting section
- 500: posture detecting section
- 502: analyzing section
- 604, 614: irradiating apparatus
- 606, 616: detector
- 700, 702, 704, 706, 708, 750, 752, 754, 756, 758: screen
- 710, 712, 760, 760a to 760e, 766: button
- 720, 722, 724, 726, 728, 730, 770, 772: message
- 740, 780: graph
- 762, 764, 764a to 764d: mark
- 790: peak
- 792, 792a to 792e: pulse wave
- 800, 802, 804: section
- 900: information processing apparatus
- 950: CPU
- 952: ROM
- 954: RAM
- 956: recording medium
- 958: input/output interface
- 960: manipulation input device
- 962: display device
- 964: audio output device
- 966: vibration generating device
- 968: communication interface
- 970: bus
- 980: sensor

## Claims

1. An information processing apparatus, comprising:
a comparing section configured to perform a comparison using a blood flow velocity of a measured person in a predetermined state and a blood flow velocity of the measured person in a state other than the predetermined state; and
an information presenting section configured to present information corresponding to a comparison result of the comparing section.

2. The information processing apparatus according to claim 1, wherein the information presenting section includes at least one of an audio output apparatus, a vibration generating apparatus, or a display apparatus.

3. The information processing apparatus according to claim 1, wherein the information presenting section presents information for guiding a user of the information processing apparatus to perform a predetermined behavior as the information corresponding to the comparison result.

4. The information processing apparatus according to claim 3, wherein the user includes the measured person.

5. The information processing apparatus according to claim 1, further comprising:
a posture detecting section configured to detect a posture of the measured person.

6. The information processing apparatus according to claim 1, further comprising:
a state detecting section configured to detect a state of the measured person.

7. The information processing apparatus according to claim 1, further comprising:
a measuring section which is installed on a measurement region of the measured person and configured to measure the blood flow velocity of the measured person.

8. The information processing apparatus according to claim 7, wherein the comparing section determines an installation state of the measuring section using a pulse wave obtained from the blood flow velocity of the measured person.

9. The information processing apparatus according to claim 7, wherein the measuring section further includes
an irradiating section configured to irradiate the measurement region with irradiation light,
a detecting section configured to detect light from the measurement region, and
a reflecting plate configured to guide the irradiation light to the measurement region or guide the light from the measurement region to the detecting section.

10. The information processing apparatus according to claim 9, wherein the irradiating section is stacked above the detecting section.

11. The information processing apparatus according to claim 9, wherein the reflecting plate includes a curved surface.

12. The information processing apparatus according to claim 9, further comprising:
a control section configured to control the irradiating section such that an irradiation pattern in which the irradiating section intermittently irradiates the irradiation light at a first interval is repeated at a second interval longer than the first interval.

13. The information processing apparatus according to claim 9, further comprising:
a control section configured to control the irradiating section such that an irradiation interval of the irradiating section is changed to a random length of time.

14. The information processing apparatus according to claim 9, further comprising:
a control section configured to control the irradiating section in accordance with an irradiation pattern corresponding to a sampling pattern obtained by combining a plurality of sampling intervals corresponding to a plurality of different detection targets.

15. An information processing method, comprising:
performing a comparison using a blood flow velocity of a measured person in a predetermined state and a blood flow velocity of the measured person in a state other than the predetermined state; and
presenting information corresponding to a comparison result obtained through the comparison.

16. A program causing a computer to implement:
a function of performing a comparison using a blood flow velocity of a measured person in a predetermined state and a blood flow velocity of the measured person in a state other than the predetermined state; and
a function of presenting information corresponding to a result of the comparison.
